# EUROPEAN PATENT APPLICATION

(11) **EP 3 798 632 A1**
(43) Date of publication of application: **31.03.2021**
(21) Application number: 19199136.3
(22) Date of filing: 24.09.2019
(51) Int. Cl.: G01N 33/50, A61K 38/00, G16H 10/00

(54) **METHODS OF IDENTIFICATION OF SYNERGISTIC ANTI-CANCER MULTIDRUG COMBINATIONS AND USES THEREOF**

(71) Applicant: UNIVERSITE DE GENEVE, 1211 Geneva 4 (CH)
(72) Inventor: NOWAK-SLIWINSKA, Patrycja, 01630 Sergy (FR); MERALDI, Patrick, 1197 Prangins (CH); WEISS, Andrea, Durham, NC 27713 (US); LE ROUX BOURDIEU, Morgan, 74100 Annemasse (FR)
(74) Representative: reuteler & cie SA

(57) **Abstract**

The present invention relates to methods for identifying multidrug combinations useful in the treatment of cancers, in particular solid tumor cancers and optimized multidrug combinations resulting therefrom. In particular, the invention relates to compositions useful in the treatment of cancer, in particular in the inhibition of spindle pole clustering in cancer cells.

## Description

### Field of the invention

The present invention relates to methods for identifying multidrug combinations useful in the treatment of cancers, in particular solid tumor cancers and optimized multidrug combinations.

### Background

Combination therapies represent an attractive treatment option due to their potential to overcome drug resistance by targeting non-overlapping signaling pathways, decreasing the probability of developing cross-resistance (Bozic et al., 2013, eLife 2:e00747)*.* While off-target toxicities are a major concern of new drug combinations, synergistic drug combinations can achieve higher therapeutic selectivity than single drugs (Lehar et al., 2009, Nat. Biotechnol. 27(7):659-66). Causally, this can be explained by various phenomena. If properly designed, synergistic drug combinations can (i) enable targeting of deregulated networks from multiple angles (Keith et al., 2005, Nat. Rev. Drug Discov., 4(1):71-78*)* thereby challenging the robust and compensatory nature of complex biological systems *(*Kitano, 2007, Nat. Rev. Drug Discov., 6(3):202-210*;* Lehár et al., 2008, Molecular systems biology 4:215-215*)* and (ii) allow for effective therapies with reduced toxicity by employing lower single drug doses of compounds with non-overlapping dose-related toxicities *(*Delaney et al., 2015, Oncotarget 6(31):31104-31118*;* Liu et al., 2016, Oncotarget, 7(10):11310-11320*).* However, a major limitation of clinically used cancer drugs is the lack of specificity resulting in toxicity.

If the use of properly tailored drug combinations is a promising alternative to overcome the above-mentioned limitations of cancer therapy, the limited success of clinically tested drug combinations, thus far, can largely be attributed to intolerable toxicities, lack of adequate efficacy and acquired drug resistance. This may be a result of current clinical practice where drug combination components are selected empirically or based on the success of single drug therapies. Subsequently, the drugs combined are not selected for their synergistic or selective activity. To address these issues, various drug optimization methods have been developed *(*Weiss et al., 2015, Sci. Rep., 5:14508*;* Weiss & Nowak-Sliwinska, 2017, SLAS Technology 22). One of the major challenges in defining optimal drug cocktails is the immense number of combinatorial possibilities. Not only must the selection of candidate drugs be taken into consideration, but also their relative dose ratios, their potential for synergisms or antagonisms, and their toxicity profiles. Already combining 10 drugs at 6 doses represents 6¹⁰, i.e. over 60 million possible drug-dose ratio combinations. Some attempts to develop methods for the identification of drug combination have been made (WO 2015/136061). However, those methods do not take toxicity into consideration, thus tending towards the identification of combinations containing more compounds at higher doses, increasing the probability of identifying combinations with increased toxicity. Furthermore, these methods employ a population-based search algorithm in order to identify drug combinations with enhanced efficacy. This method requires the evaluation of a greater number of drug combinations as well as more than ten iterative search rounds, slowing the speed and cost of optimization searches.
Moreover, the 'one size fits all' approach of cancer therapy, where patients are given a treatment based on their cancer type or, in the best case, based on a common genetic marker (or mutant oncogene), remains unsatisfactory. For example, most commonly used drugs to treat advanced RCC are agents that target vascular endothelial growth factor (VEGF) receptors and those that inhibit mTOR. However, while most patients initially respond to these agents with time, they develop resistance and subsequent disease progression.

Abnormalities in centrosome organization such as supplementary centrosome number are very frequent in cancer cells (Prakash et al., 2018, Molecules 23, doi:10.3390/molecules23051166). This excessive number of centrosomes leads to multipolar cell division that in turn leads to cell death due to massive chromosome mis-segregation *(*Godinho et al., 2014, Biological sciences, 369, doi:10.1098/rstb.2013.0467). Cancer cells elaborated a survival mechanism to overcome that problem called centrosome clustering (CC). In this process supplementary centrosomes are clustered onto two spindle poles during the assembly of the mitotic spindle, resulting into a pseudo-bipolar cell division. Centrosome clustering only leads to a mild rate of chromosome loss/gain, allowing cells to survive (Kramer et al., 2011, Mol Oncol 5, 324-335, doi:10.1016/j.molonc.2011.05.003). Proof-of-principle experiments based on siRNA depletions of key microtubule motors required for centrosome clustering showed that impairing centrosome clustering causes multipolar cell death that are associated with cell death in the ensuing interphase (*Godinho et al., 2014, supra*)*.* Importantly, these treatments did not affect cell viability in non-cancerous cells with a normal number of centrosomes. Therefore, drugs impairing centrosome clustering appear to be an attractive novel approach in oncology.

Centrosome clustering inhibitors represent an attractive anti-cancer strategy, as they should be inactive in non-transformed cells. The currently available inhibitors targeting centrosome clustering were recently reviewed (*Prakash et al., 2018, supra*)*.* Well-known examples include taxol (Paclitaxel) or epothilone B that induces multipolar spindles (Zasadil et al., 2014, Science translational medicine 6, 229ra243, doi:10.1126/scitranslmed.3007965*;* Chen and Horwitz, 2002, Cancer Research 62, 1935-8) and crenolanib, an anti-PDGFR compound that has been reported to present centrosome clustering (Konotop et al., 2016, Cancer research 76, 6690-6700, doi:10.1158/0008-5472.CAN-16-1144). Unfortunately, their use leads to acquired resistance and severe side effects in cancer patients, as they also target other biological processes.

Therefore, there is unmet need for finding new, safer CC inhibitors that would overcome these shortcomings and to develop effective combination therapy for cancer treatment, in particular for unmet medical needs in the treatment of solid tumors.

### Summary of the Invention

The invention is based on the unexpected findings that a phenotypically driven screening method based on a Therapeutically Guided Multidrug Optimization (TGMO) technique where drug combinations are tested simultaneously on both cancer (e.g. ccRCC cells (786-O)) and non-cancer cells (e.g. non-malignant renal cells) at low doses (i.e. below maximal plasma doses (MPD) in humans) allows to identified identify optimal multidrug combinations acting with high efficacy and selectivity (e.g. in clear cell renal cell carcinoma (ccRCC)). This newly developed method was validated with the development of a four-drug combination which has been shown to be efficient in inhibiting multipolar spindle pole clustering, a survival mechanism employed by cancer cells with spindle abnormalities.
Therefore, the invention further relates to a newly developed combination therapy which is surprisingly highly effective and selective for the treatment of malignant and resistant cancers such as clear cell renal cell carcinomas and would be useful in the preventing of centrosome clustering.
Therefore, it was unexpectedly found that a method based on the use of response surface methodology and the design of experiment (DoE) approach with linear regression analysis which selectively samples a minimal number of experimental data points in order to create the cell's response surface, i.e. drug dose-efficacy dependence in terms of second-order linear regression models that are used to select synergistic drug interactions powerfully allows to identify optimal multidrug combinations acting with efficacy and selectivity higher than the first line treatment after testing only 0.37% of all possible experimental conditions.

According to one aspect of the invention, is provided a method for identifying low-dose multi-drug combinations for the treatment of a cancer, in particular solid tumor cancers and uses thereof for the treatment of a cancer.
According to another aspect of the invention, is provided a combination of CI-994, tubacin, erlotinib and dasatinib for use in the treatment of a cancer.
According to another aspect of the invention, is provided a use of a combination of CI-994, tubacin, erlotinib and dasatinib for the preparation of a pharmaceutical composition for the treatment of cancer.
According to another aspect of the invention, is provided a pharmaceutical composition comprising a combination of the invention and at least one pharmaceutically acceptable carrier and its use as a medication.
According to another aspect of the invention, is provided a method for the treating a subject who is suffering from a cancer, said method comprising the administration of a therapeutically effective amount of a combination according to the invention or any suitable pharmaceutically acceptable formulation thereof, in a subject in need thereof.
In another embodiment, the invention provides a method of preventing the formation of centrosome clustering in cancer cells, said method comprising the administration of a therapeutically effective amount of a combination of the invention or any suitable pharmaceutically acceptable formulation thereof, in a subject suffering from a cancer, in particular a solid tumor cancer.
In another embodiment, the invention provides a method of preventing the resolution of multipolar spindles induced by anti-cancer drugs, such as paclitaxel or epothilone B, said method comprising the administration of therapeutically effective amount of a combination of the invention or any suitable pharmaceutically acceptable formulation thereof in further combination with at least on one anti-cancer drug inducing multipolar spindles (e.g. paclitaxel or epothilone), in a subject suffering from a cancer, in particular a solid human cancer.

### Description of the figures

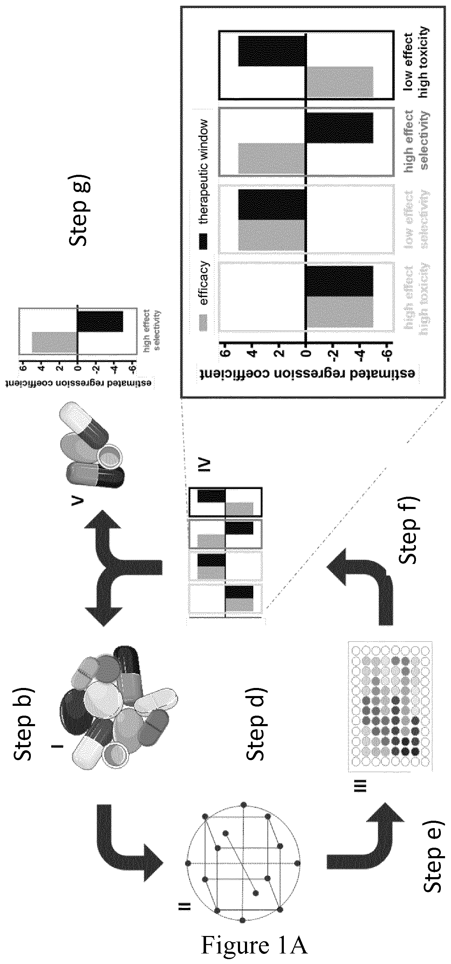

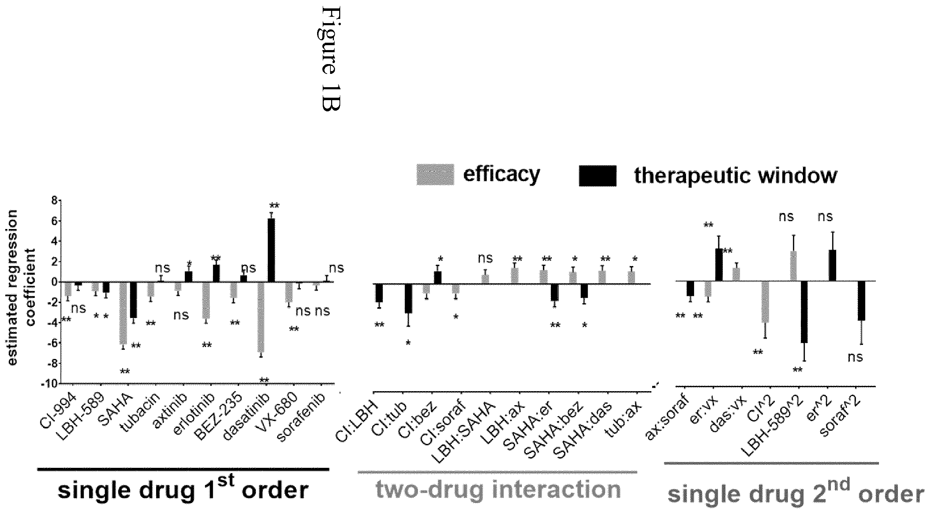

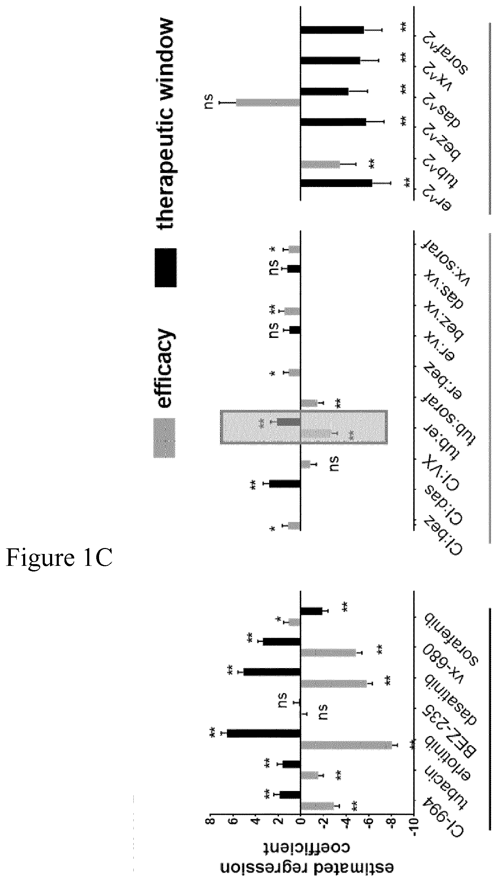

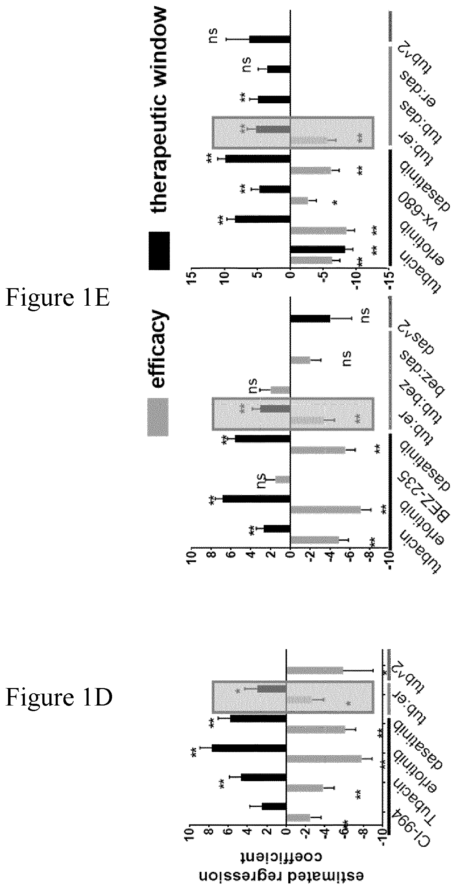

**Figure 1** is an overview of a method of identifying a drug combination according to the invention as illustrated under Example 1. **A:** Schematic overview of the method where an initial set of candidate compounds was selected (e.g. ten) **(I):** Dose-response curves for each of the selected candidate compounds were generated for each cell type (malignant and non-malignant) using inhibition of metabolic activity as a measure for cell viability as described under steps a) to c) of a method according to the invention; **(II):** Defining a compound candidate combination matrix to be tested via orthogonal array composite design (OACD) as described under step d) of a method according to the invention; **(III):** Assessing the difference in efficacy between non-malignant embryonic kidney 293 (HEK-293T) cells and malignant 786-O cells (in grey) of each candidate compound combination, thereby determining the therapeutic window (in black) as described under step e) of a method according to the invention; **(IV):** Modeling of both the efficacy in 786-O cells and the therapeutic window using a step-wise second-order linear regression model as described under step f) of a method according to the invention: second-order linear regression models are used to describe the relationship between the response variable (e.g. cell metabolic activity inhibition) and the input (compound) of compound combinations (i.e. the compounds and corresponding compound doses administered) where regression coefficients describe the contribution of each individual drug and drug pair on the change in the combinations activity in terms of the response variable; **(V)** Selecting candidate compound combinations (exhibiting an optimal activity profile, being both effective and non-toxic) based on the obtained regression coefficients as described under step g) of a method according to the invention; Iterative refined data analysis steps b) to g) are performed where experimental data is used to develop regression models used to analyse drug interactions and eliminate candidate compounds (right arrow) before returning to the closed-loop optimization protocol (left arrow), allowing for the final compound combination selection after three sequential searches (experimental DoE/OACD-based screen). **B to E:** represent the estimated regression coefficients resulting from: **(B)** from the modelling in the first iteration step f) on the initial compound combination set; **(C** and **D)** from the modelling in the sub-sequent iteration steps (respectively f1 and f2) made on the compound combination sets resulting from the earlier iteration step f) where generally three compounds are eliminated from the combination for the next iteration step, based on lack of compound activity or antagonistic compound interactions (f1: iteration step on a 7-compound combination and f2: iteration step on a 4-compound combination), and resulting from the modelling in the first iteration step f) on the third compound combination set; **(E)** from models of efficacy in 786-O cells represented in grey and the therapeutic window models presented in black. Grey boxes highlight the most relevant synergistic activity consistent throughout the sequential searches and resulted in the selection of the optimal combination. Significance is represented with *p < 0.05 and **p < 0.01.

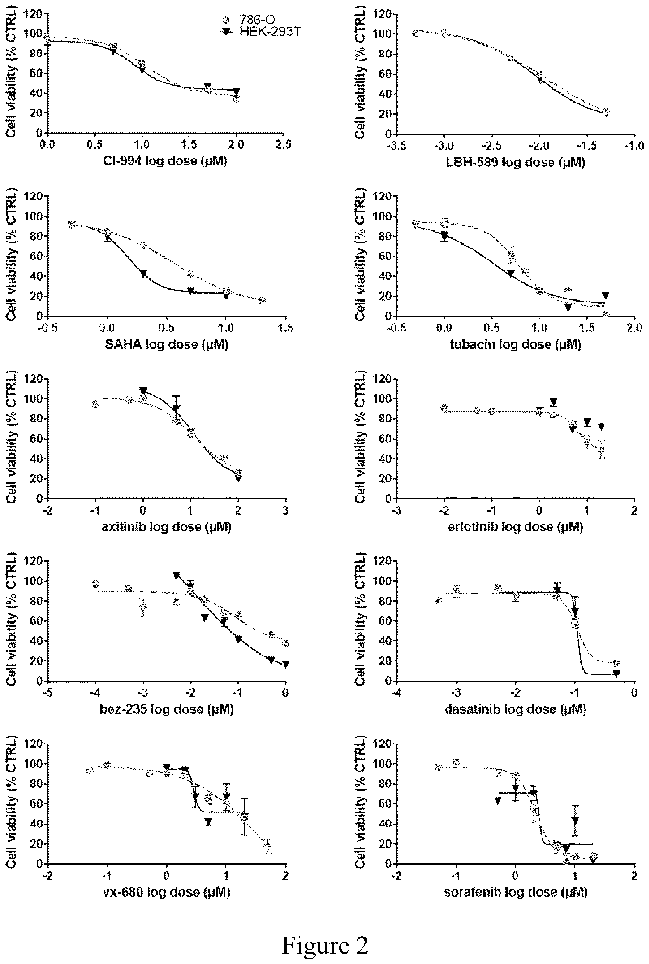

**Figure 2** shows 786-O and HEK-293T dose response curves for all individual candidate compounds performed under step c) of a method of the invention as illustrated under Example 1.

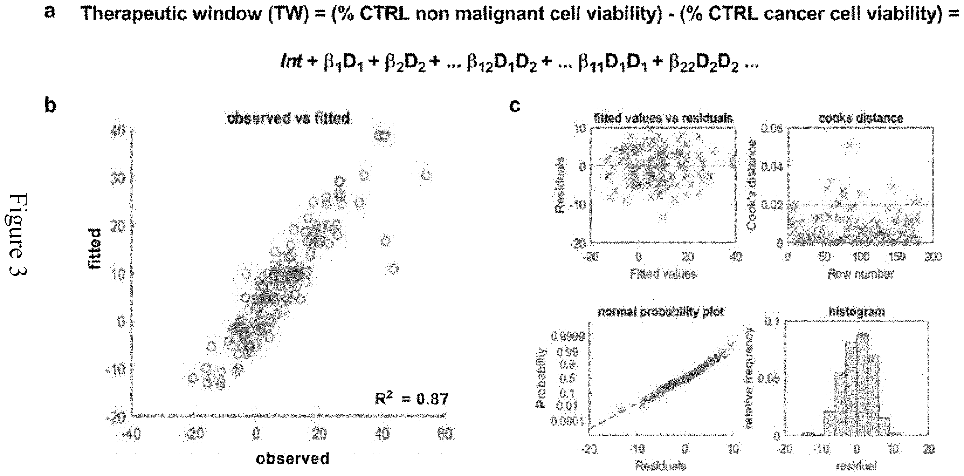

**Figure 3** illustrates steps e) and f) of a method of the invention as illustrated under Example 1 under first iteration round: **a:** defines the determination of the therapeutic window (TW) of each compound candidate combination based on the difference in efficacy in cell metabolic activity inhibition - indirectly cell viability inhibition - between the malignant and non-malignant cells as performed under step e) of a method of the invention. **b:** Plot of observed (i.e. experimental data points) vs. fitted values (i.e. activity predicted by the linear regression models) showing the R²-value (coefficient of determination) as an indicator of goodness of fit; **c:** Residual plots used for model analysis and the elimination of outlier data points if present (based on Cook's distance, generally defined as points with Cook's distance greater than three times the average cook's distance of all data points). Residual plots include the observed vs. fitted data points, residual plot, Cook's distance plot, normal probability plot and residual histogram.

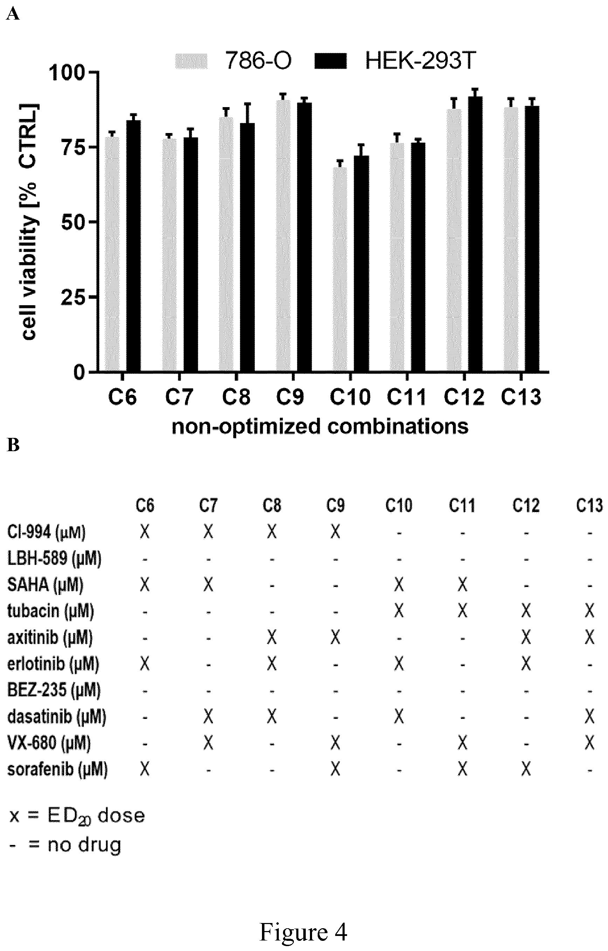

**Figure 4** represents the efficacy of random non-optimized 4-drug combinations as part of the initial design matrix screened in step f1) **(A)** and composition of corresponding drug combinations **(B).**

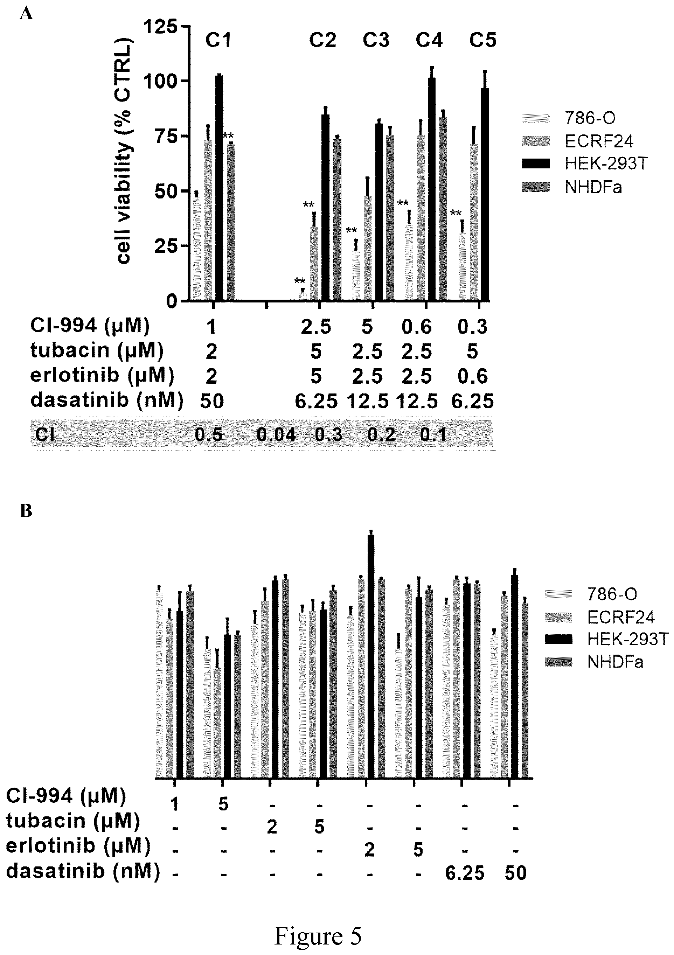

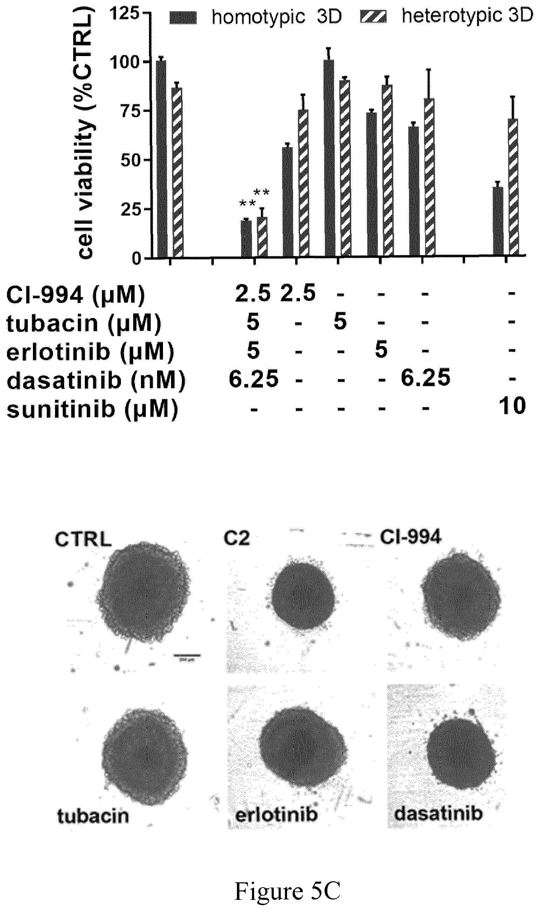

**Figure 5** shows the dose optimization and validation of the optimized drug combination **C1** in 3D cell cultures and sunitinib resistant cells as described in Examples 1 and 2. **A:** Efficacy of the five most promising drug combinations (**C1-C5**) derived from the dose optimization with **C1** evaluated on the basis of cell viability for the 786-O cell line, non-malignant embryonic kidney 293 (HEK-293T) control cells and non-malignant NHDFa fibroblasts and activated ECRF24 endothelial cells. 'Combination index' (CI) values for each drug combination with CI<1 indicating synergy, 0 and CI>1 indicating antagonism. *p < 0.05 and **p < 0.01 represent significances compared to all corresponding single drug treatments as determined by a one-way ANOVA with post-hoc Tukey's multiple comparison test. **C:** Efficacy (top) and representative images (bottom) of the dose-optimized drug combination **C2** in 3D homotypic (786-O) spheroids or in 3D co-culture heterotypic spheroids containing human fibroblasts, 786-O (1:1) and 10% ECRF24 endothelial cells. Sunitinib at 10 µM was used as a positive control. Scale bar represents 200 µm for all images.

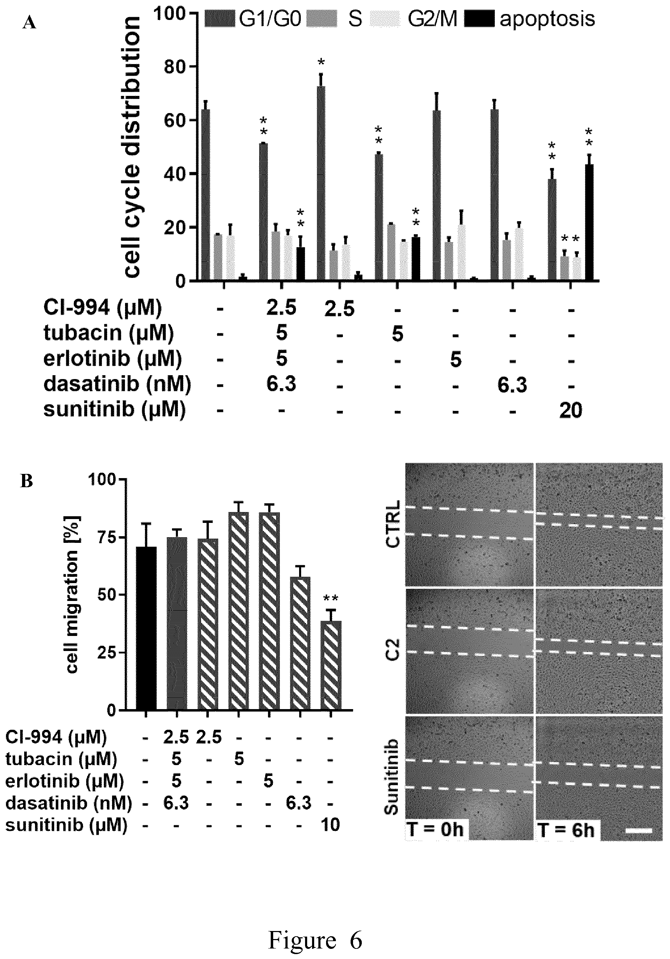

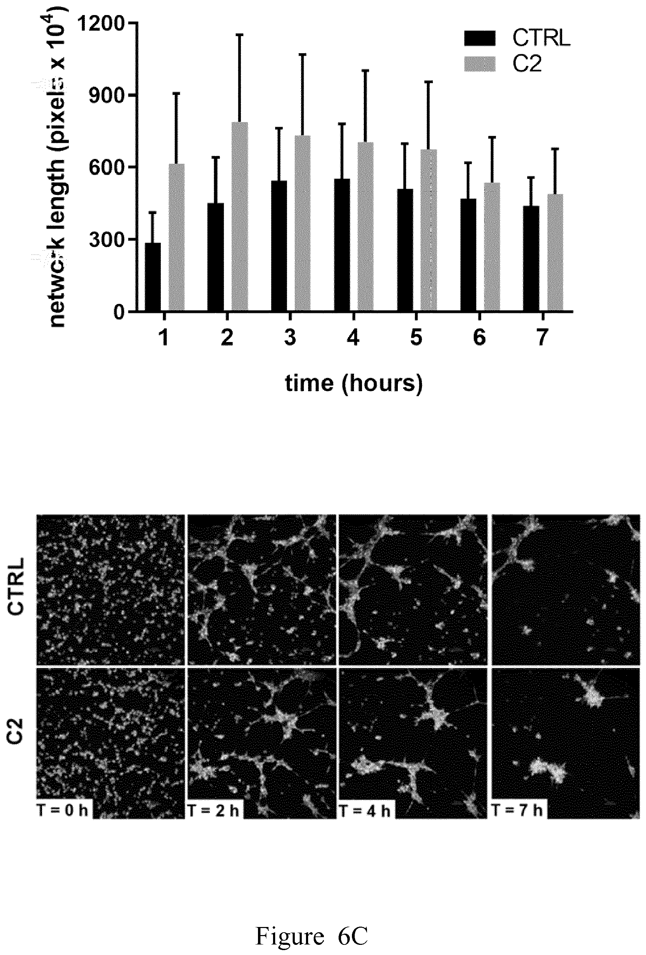

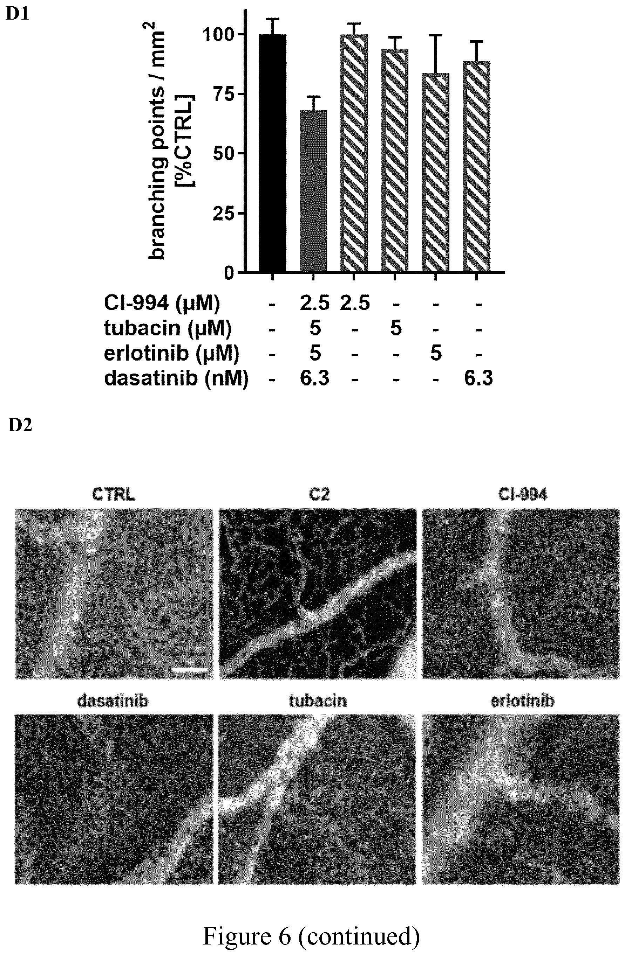

**Figure 6** represents the influence of **C2** treatment on 786-O cell cycle and cell morphology influenced as described in Example 3. **A:** Cell cycle analysis of ECRF24 cells treated with the optimized drug combination for 72h. Percentage of cells in G1, S, G2/M or sub-G1 (apoptotic) is indicated compared to the 0.1% DMSO CTRL. Bars represent the mean of 2 independent experiments performed. *p < 0.05 and **p < 0.01 represent significance versus CTRL determined by a two-way ANOVA with post-hoc Dunnett's multiple comparison test. **B:** 2D migration in ECRF24 cells where the measured percentage of cell migration is following the administration of a 'scratch' wound and 7 hours of incubation with the **C2** drug combination, corresponding monotherapies and sunitinib (positive control) (left). **p < 0.01 represents significance versus CTRL as determined by a one-way ANOVA with post-hoc Dunett's multiple comparison test from two independent experiments with n=6. Right: Representative images of 2D migration assay are shown for CTRL, **C2** and sunitinib at the beginning (T=0) and after 5 hours incubation with the drugs (T=5). Scale bar corresponds to 400 µm; **C:** network length measured in a matrigel co-culture endothelial network formation assay with human umbilical vein endothelial cells (HUVEC) showing endothelial network formation over 7 hours and confocal images of cells showing the formation of an endothelial network in the CTRL treated wells. Quantification of the total endothelial network length in Fiji/ImageJ with Angiogenesis Analyzer shows no significant inhibition of HUVEC network alignment and network formation (mean of two experiments with n=6); **D:** *In vivo* inhibition of angiogenesis in the developmental angiogenesis evaluated in the chorioallantoic membrane (the CAM) model of the chicken embryo following two consecutive days of topical administration of the **C2** combination (**D1**). Inhibition of capillary growth in CAMs treated with **C2** as presented by image-based quantification of the number of branching points/mm3 (**D2**). **p < 0.01 represents significance versus CTRL (mean of minimally 2 experiments with n=4-15) as determined by a one-way ANOVA with posthoc Sidak's multiple comparison test. Error bars represent ± SEM.

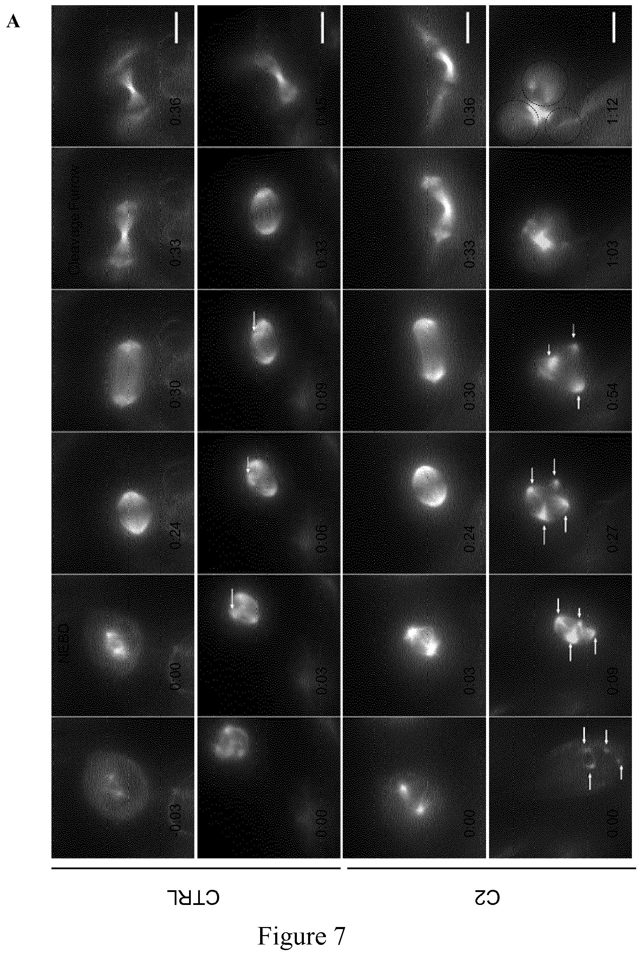

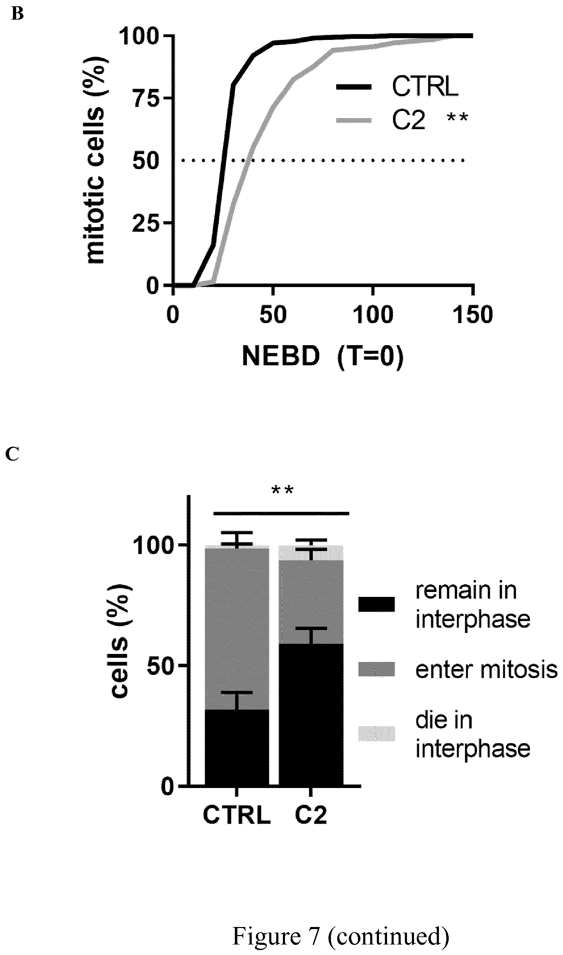

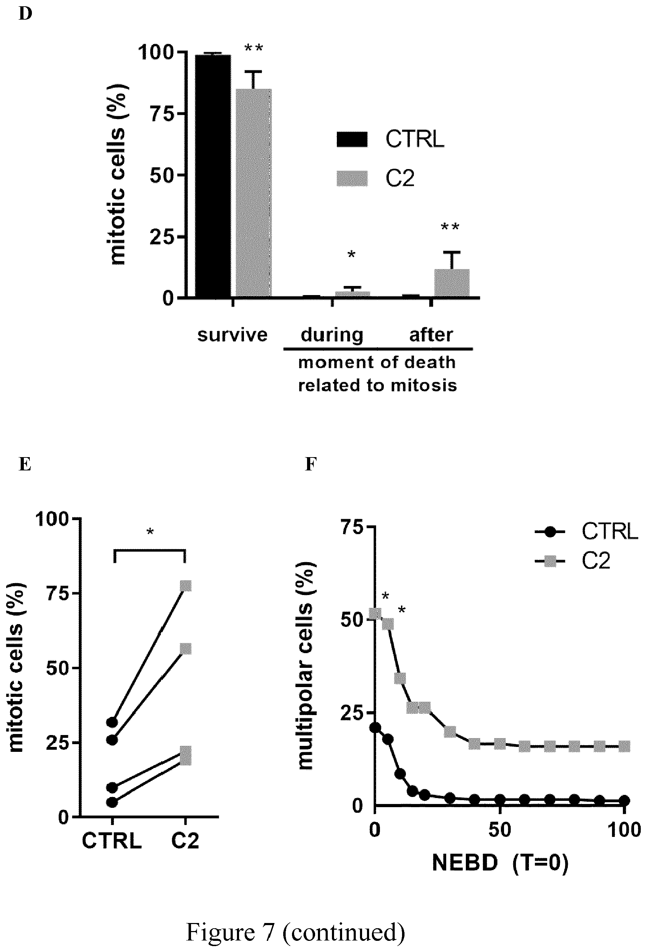

**Figure 7** shows the activity of **C2** combination on centrosome clustering in 786-O cells as described in Example 5. **A:** Time-lapse images of CTRL (0.1% DMSO) or **C2**-treated 786-O cells undergoing mitosis, stained with SiR-Tubulin. Mitotic timing in h:mins using nuclear envelope breakdown (NEBD) as T = 0. The arrows in the **C2**-treated multipolar cells indicate spindle poles, dashed circles the daughter cells after mitotic completion. Scale bar represents 10 µm for all images. **B:** Mitotic timing is defined as the time NEBD until formation of a cleavage furrow. Significances of **p < 0.001 compared to CTRL as determined by a in a Mann-Whitney Test from n = 3 experiments with n = 130-294 cells. **C:** Outcomes of 786-O cells treated with CTRL (0.1% DMSO) or C2 during the 24h movies. Significance of **p<0.0001 compared to CTRL as determined by a Fisher's exact test, N = 4 independent experiments with a total of n = 606 (CTRL) and 603 (C2) cells. **D:** Quantification of mitotic outcomes of 786-O cells, (normal mitosis, death in the ensuing interphase and death in mitosis), treated with CTRL (0.1% DMSO) or **C2.** Significance of **p<0.0001 and *p<0.05 compared to CTRL as determined by a Fisher's exact test, N = 4, n = 406 (CTRL) and 206 (C2). **E:** Percentage of multipolar cells in the individual experiments (each percentage is compared to its paired, control experiment. *p<0.05 compared to CTRL as determined by a paired two-tailed t-test with from N = 4 independent experiments; n = 406 (CTRL) and 206 (C2) cells **F:** Percentage of multipolar cells that cluster its spindle poles. Significances of *p = 0.0144 and *p = 0.0129 for the first two points compared to CTRL as determined by an in two-way ANOVA with Sidak's multiple comparisons test from n = 3 experiments with n = 128-223 and n = 59-106 cells for CTRL and **C2**-treated cells, respectively. Error bars represent ± the standard error of the mean (SEM) for all graphs.

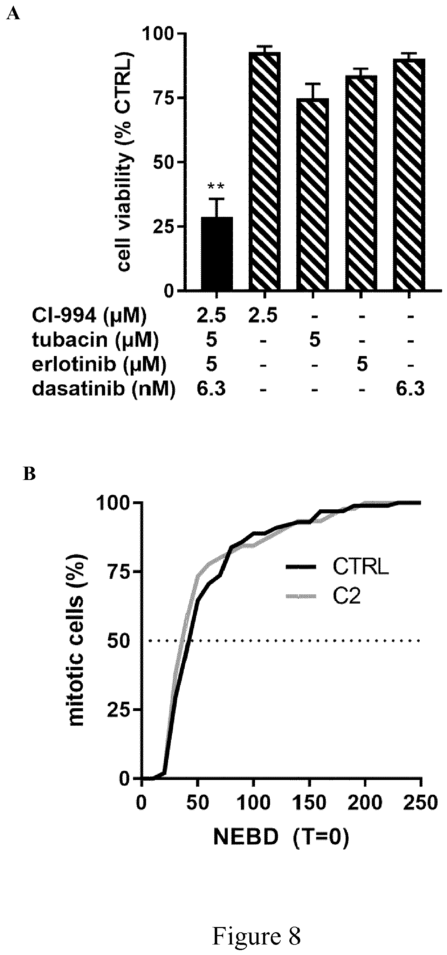

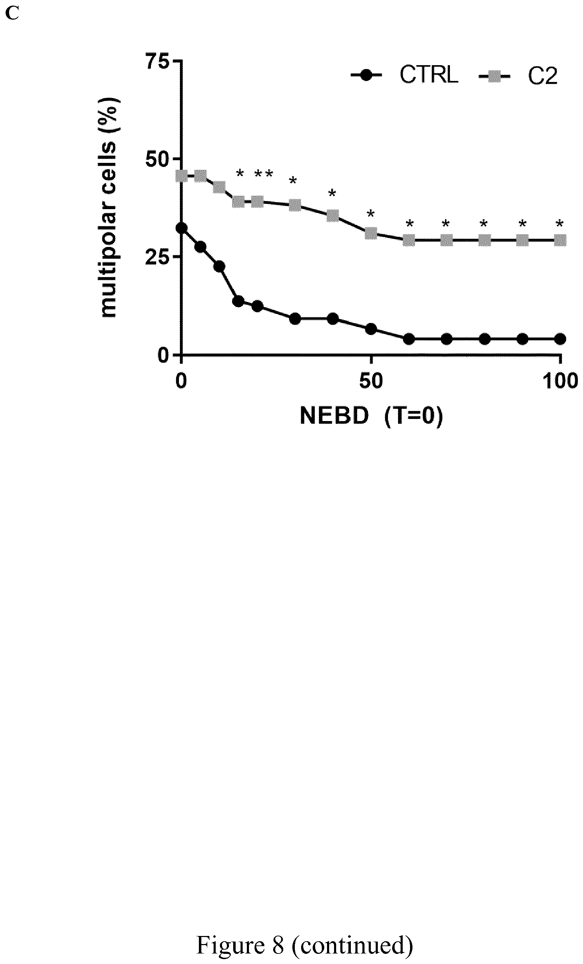

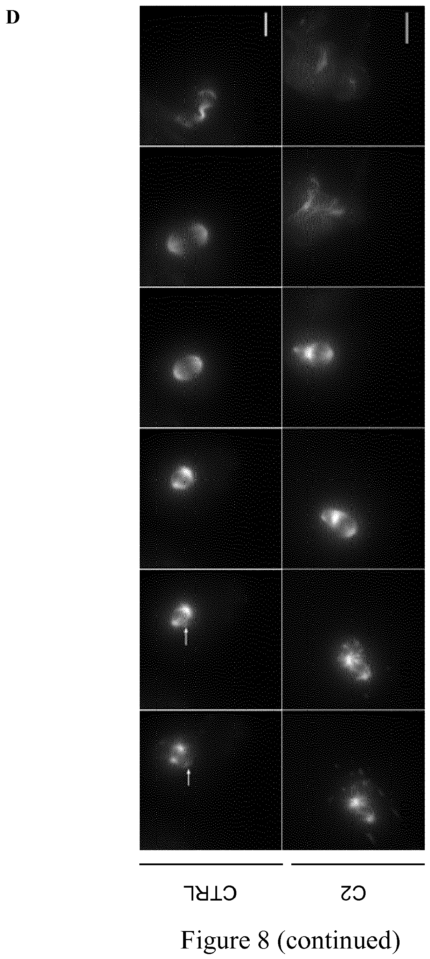

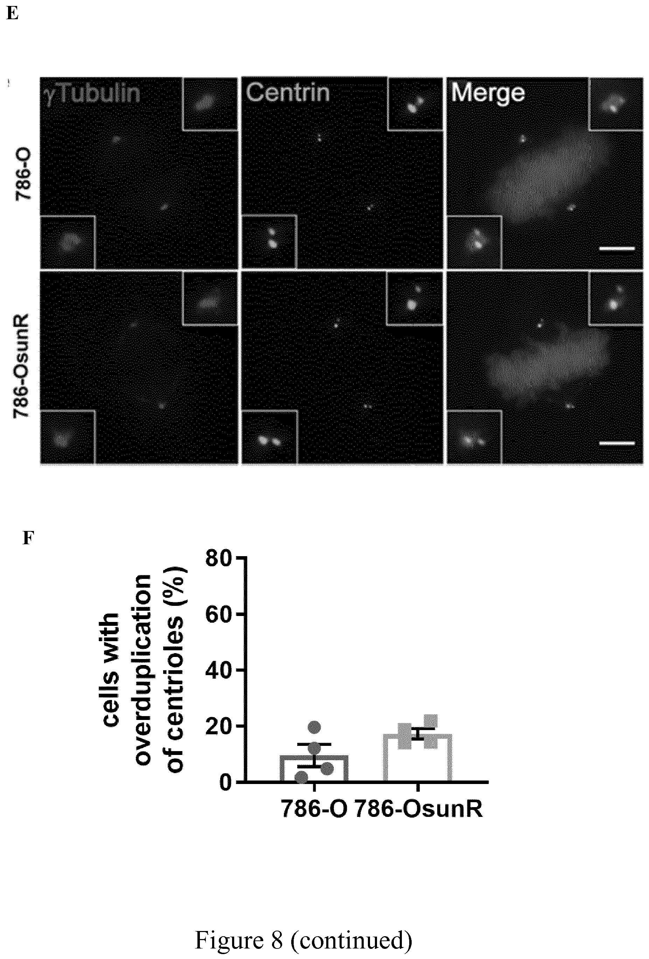

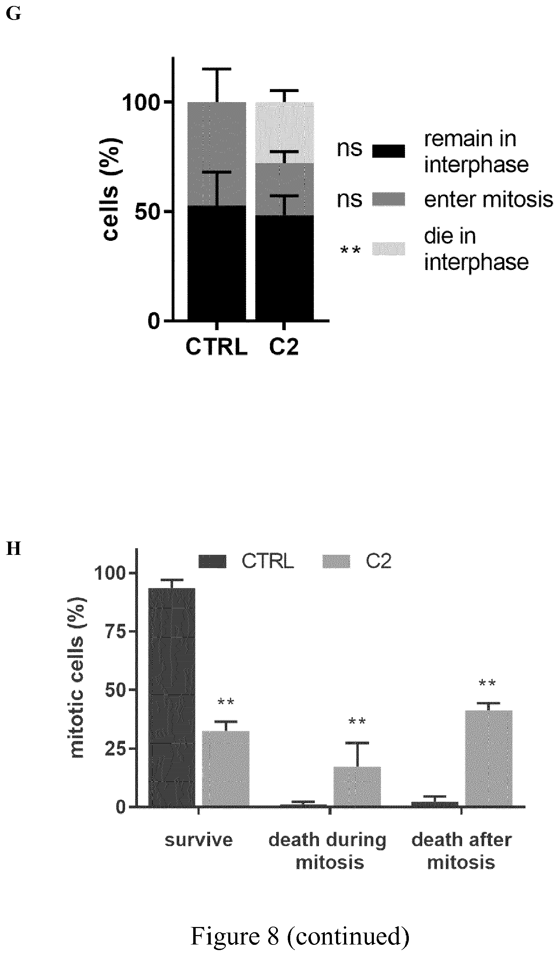

**Figure 8** represents the activity of **C2** on spindle pole clustering in sunitinib-resistant 786-O cells. **A:** Efficacy of C1 and C2 and corresponding single drug treatments in 786-O cells chronically exposed to sunitinib treatment (786-OsunR). Bars represent the mean of at least 2 independent experiments performed in triplicate (n = 6). *p < 0.05 and **p < 0.01 represent significances versus all corresponding single drug treatments as determined by a one-way ANOVA with post-hoc Tukey's multiple comparison test. **B:** Mitotic timing is defined as the time of nuclear envelope breakdown (NEBD) until formation of a cleavage furrow. Significances of p = 0.2262 compared to CTRL (0.1% DMSO) as determined by a Mann-Whitney Test from n = 3 experiments with n = 45-99 cells. C: Percentage of multipolar cells that cluster its spindle poles. Significances of *p < 0.05 and **p < 0.005 compared to CTRL group as determined by an in two-way ANOVA with Sidak's multiple comparisons test from n = 3 experiments with n = 63-101 cells for CTRL and **C2**-treated cells. **D:** Time-lapse images of CTRL and C2-treated 786-OsunR cells stained with SiR-Tubulin. Mitotic timing in h:mins using NEBD as T = 0. The arrows in the CTRL multipolar MDA435 cells indicate spindle poles. Scale bar represents 10 µm for all images. **E:** Representative images of 786-O and 786-OsunR cells stained for centrin, γ-tubulin and DAPI. Scale bar represents 5 µm for all images. **F:** Image-based quantification was performed on the number of centrioles per cell. Results from n = 3 experiments with n = 196-172 cells for 786-O and 786-OsunR cells. **G:** . Outcomes of 786-OsunR cells treated with CTRL or **C2** during the 24H movie. Significance of **p<0.0001 compared to CTRL as determined by a Fisher's exact test, N = 3 independent experiments with n = 221 (CTRL) and 259 (C2) cells. **H:** Mitotic outcome of 786-O-sunR cells. Significance of **p<0.0001 compared to CTRL as determined by a Fisher's exact test, N = 3 independent experiments with n = 106 (CTRL) and 65 (C2) cells. Error bars represent ± the standard error of the mean (SEM) in all graphs.

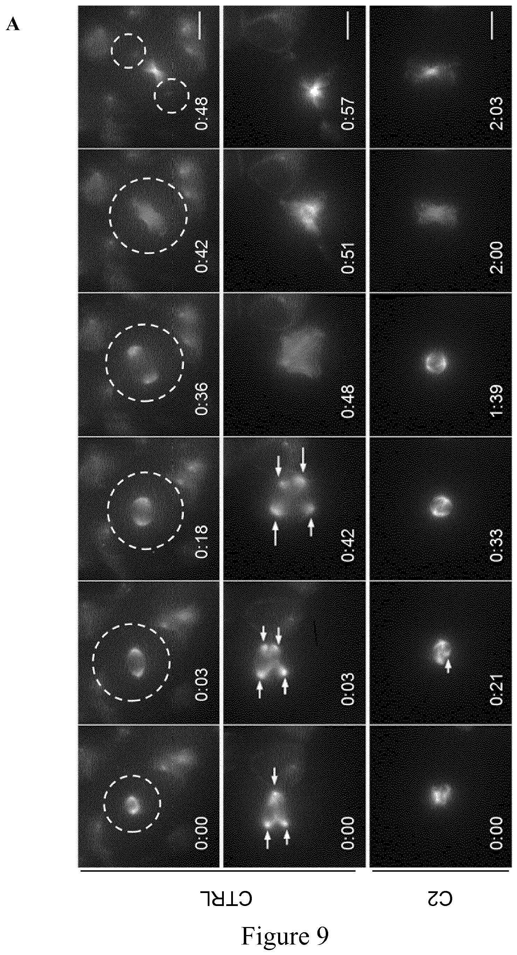

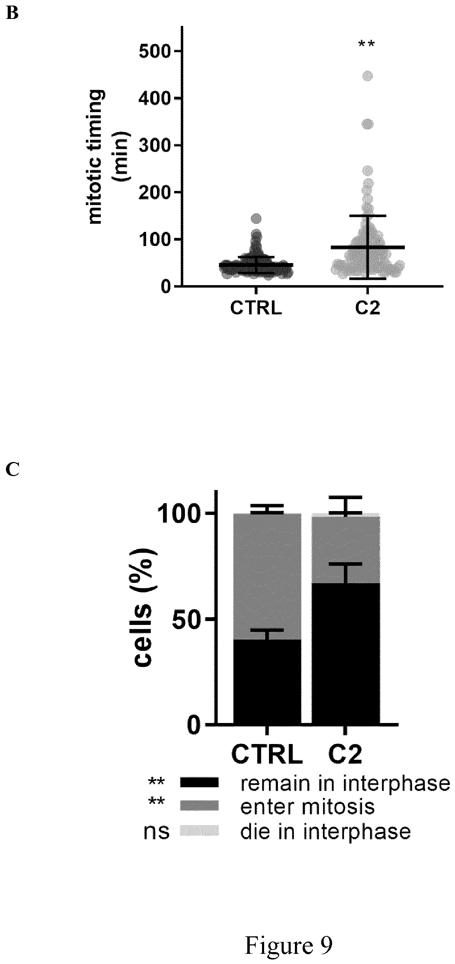

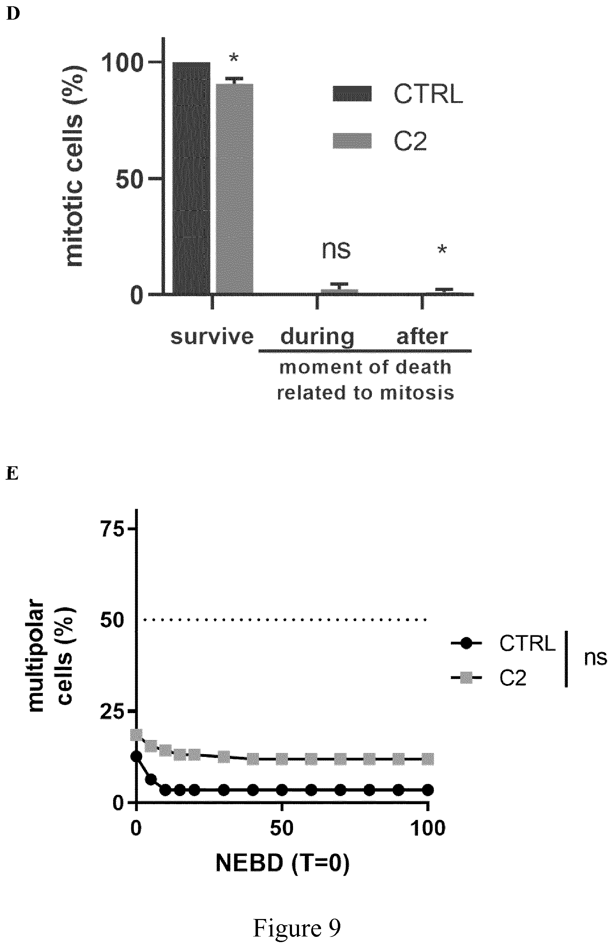

**Figure 9** represents the phenotype observed on HEK cells treated with **C2. A:** Time-lapse images of CTRL and **C2-**treated HEK cells stained with SiR-Tubulin. Mitotic timing in h:mins using NEBD as T = 0. The arrows in the CTRL multipolar HEK cells indicate spindle poles. Scale bar represents 10 µm for all images. **B:** Mitotic timing of HEK treated with CTRL or **C2.** Significances of **p<0.0001 compared to CTRL (0.1% DMSO) as determined by a Mann-Whitney Test from n = 2 experiments with n = 111-131 cells. C: Outcomes of HEK cells treated with CTRL or **C2** during 24h movies. Significance of **p<0.0001 compared to CTRL as determined by a Fisher's exact test, N = 2 independent experiments with n = 217 (CTRL) and 327 (C2) cells. **D:** Mitotic outcome of HEK cells. Significances of *p < 0.05 compared to CTRL as determined by a Fisher's exact test from N = 2 independent experiments with n = 131 (CTRL) and 111 (C2) cells. **E:** Percentage of multipolar cells that cluster its spindle poles. **C2**-treated cells were compared to CTRL group as determined by an in two-way ANOVA with Sidak's multiple comparisons test from n = 2 experiments with n = 131-111 cells for CTRL and **C2**-treated cells.

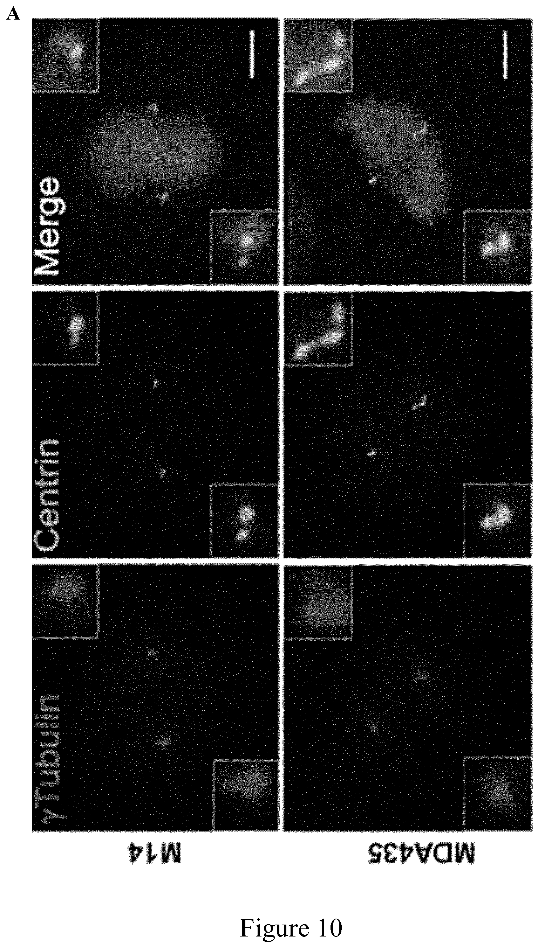

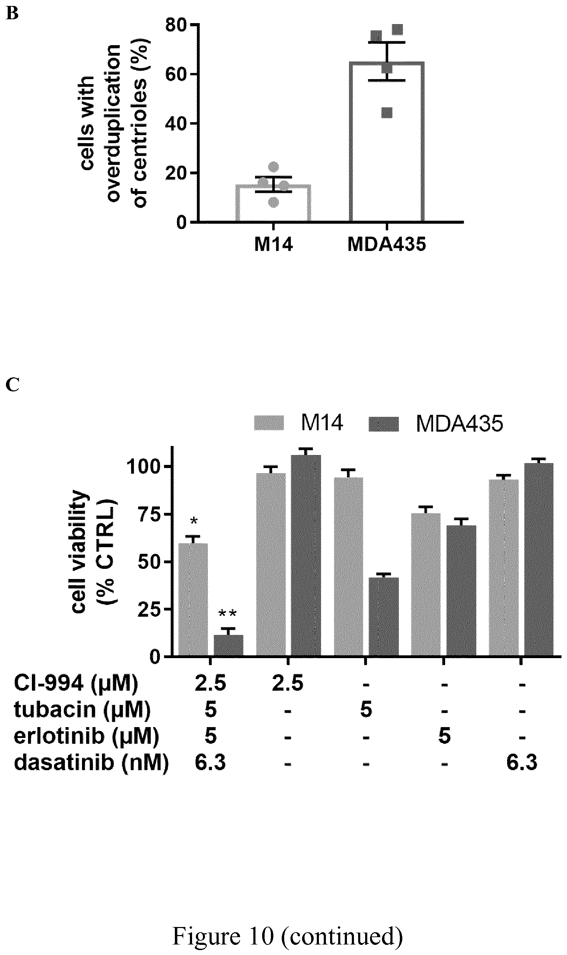

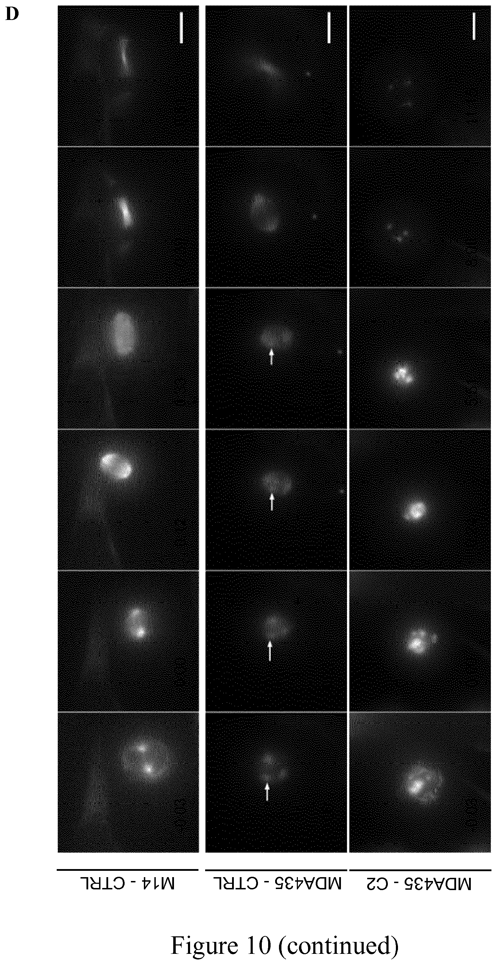

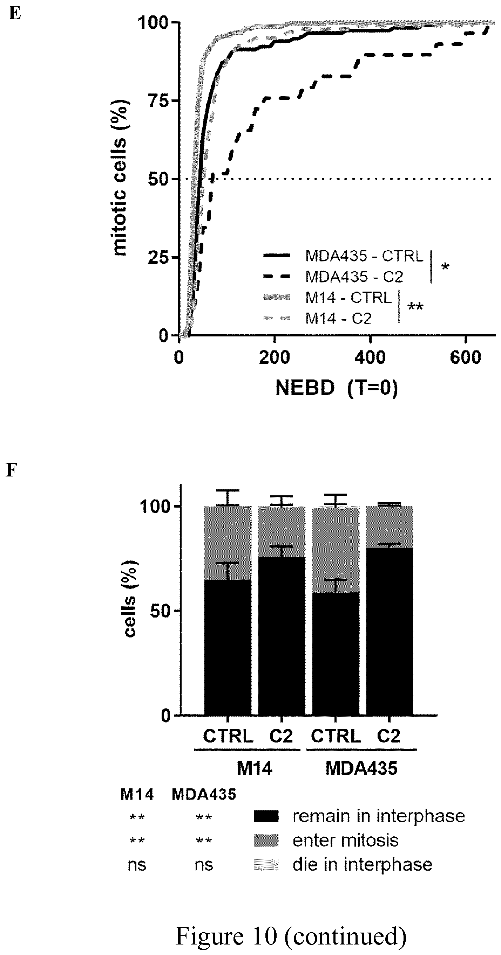

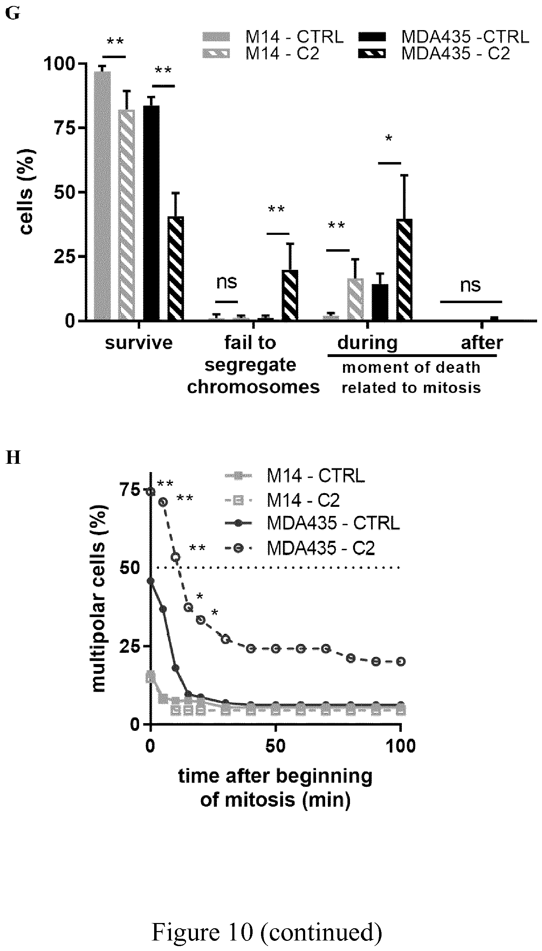

**Figure 10** represents the activity and mechanism of action of **C2** on low (M14) or high (MDA435) number of extra centrosomes. **A:** Representative immunofluorescent images of M14 and MDA435 cells stained for centrin, γ-tubulin and DAPI. Scale bar represents 5 µm for all images. **B:** Image-4-based quantification of number of centrioles per cells. Results from n = 4 experiments with n = 165-164 cells for M14 and MDA435 cells, respectively. **C:** Efficacy of **C2** and corresponding single drug treatments in M14 and MDA435 cells, *p < 0.01 and **p < 0.001 represent significance versus all corresponding single drug treatments as determined by a one-way ANOVA with post-hoc Tukey's multiple comparison test. **D:** Time-lapse images of M14 and MDA435 cells undergoing mitosis, stained with SiR-Tubulin. Mitotic timing in h:mins using nuclear envelope breakdown (NEBD) as T = 0. The arrows indicate spindle poles in the **C2**-treated multipolar MDA435 cells. Scale bar represents 10 µm for all images. **E:** Mitotic timing is defined as the time of NEBD until formation of a cleavage furrow. Significances of **p <0.0001 for M14 cells compared to CTRL (0.1% DMSO) as determined by a Mann-Whitney Test from n = 3 experiments with n = 29-221 cells **F:** Outcomes of M14 and MDA435 cells treated with CTRL or **C2** during 24h movies. Significances of **p < 0.0001 compared to CTRL as determined by a Fisher's exact test, n = 610-515 cells for M14 and n=355-297 cells for MDA435, for CTRL and **C2**-treated cells respectively. **G:** Mitotic outcome of M14 and MDA435 cells. Significances of **p < 0.0001 and *p < 0.001 compared to CTRL as determined by a Fisher's exact test, n = 3 experiments with n = 227-122 cells for M14 and n=140-60 cells for MDA435, for CTRL and **C2**-treated cells respectively. **H:** Percentage of multipolar cells that cluster its spindle poles. Significances of *p < 0.05 and **p < 0.01 compared to CTRL as determined by a two-way ANOVA with Sidak's multiple comparisons test from n = 3 experiments with n = 128-223 and n = 59-106 cells for CTRL and **C2**-treated MDA435 and M14 cells, respectively. Error bars represent ± the standard error of the mean (SEM) in all graphs.

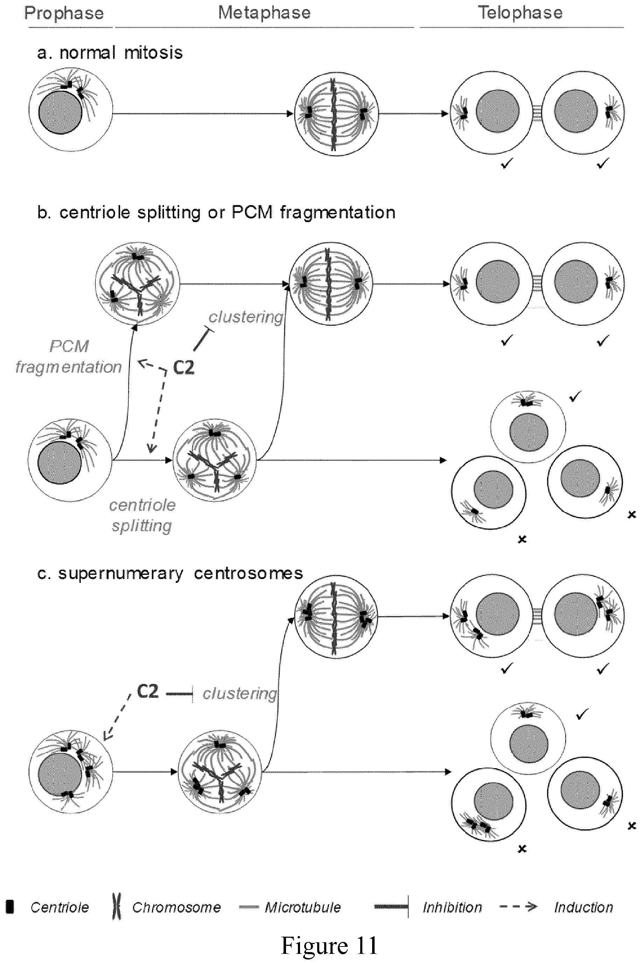

**Figure 11** illustrates the various phenotypes triggering multipolarity in mitosis. **A:** Illustration of a normal bipolar mitosis (Cancer cells can pass through a transient multipolar state before clustering its extra poles to divide as a bipolar spindle, which increases their survival rate. This transient multipolarity can be caused either by cells losing the integrity of their centrosomes by centriole splitting of PCM fragmentation **(B),** or cells already have extra centrosomes before starting mitosis **(C).** If the clustering is prevented, the daughter cells will be aneuploid and this can lead to cell death through apoptosis.

### Detailed description

The expression "pharmaceutically acceptable salts or complexes" refers to salts or complexes of the below-specified compounds. Examples of such salts include, but are not restricted, to base addition salts formed by reaction of those compounds with organic or inorganic bases or inorganic acids (e.g. hydrochloric acid and the like).

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a melanoma in a mammal, particularly a human, and includes inhibiting the disease, i.e., arresting its development; or relieving the disease, i.e., causing regression of the disease and/or its symptoms or conditions such as improvement or remediation of damage.

The term "efficacy" of a treatment or method according to the invention can be measured based on changes in the course of disease or condition in response to a use or a method according to the invention. According to a particular embodiment, the efficacy of a combined use according to the invention can be measured through the assessment of efficacy of a compound combination of the invention, in particular long term efficacy regarding repression of tumor growth, progression and dissemination, decrease of the number of cancer cells or their proliferation rate as compared to the efficacy of each compound of the combination, taken alone. According to another embodiment, the efficacy of a combined use according to the invention can be assessed by the observation of a decrease or inhibition of the emergence of an adaptive resistance of cancer cells under treatment with a single compound, for example by clinical observation of increased progression free survival or overall survival.

According to another aspect, the efficacy of a combined use according to the invention can be assessed through the observation by light microscopy techniques in cancer cells of a cell cycle arrest, actin cytoskeleton disruption, and dysfunctional cell divisions due to persistent aberrant mitotic spindle structures. Multipolar spindles can be caused by the presence of more than two centrosomes, centriole dis-engagement, centriole breakages or the fragmentation of the peri-centriolar material and therefore the maintenance of persistent multipolar spindles by combinations of the invention can be monitored.

The term "subject" as used herein refers to mammals. For examples, mammals contemplated by the present invention include human, primates, domesticated animals such as cattle, sheep, pigs, horses, laboratory rodents, other pets and the like.

### Method of identifying synergistic drug combinations according to the invention

According to one aspect, is provided a method for identifying low-dose multi-drug combinations for the treatment of cancer, in particular solid tumor cancers, said method comprising the steps of:
**a)** providing separately a set of cancer cells (malignant) and a set of non-cancer cells (non-malignant);
**b)** selecting an initial set of candidate compounds to be assayed;
**c)** contacting said set of cancer cells and a set of non-cancer cells with each of the selected candidate compound and measuring metabolic activity of said cells for different concentrations of candidate compound thereby generating dose-response curves for each candidate compound;
**d)** designing a candidate compound combination matrix by orthogonal array composite design (OACD);
**e)** contacting a new set of cancer cells and a new set of non-cancer cells with each of the candidate compound combinations designed under step d) and measuring the difference in anti-cancer efficacy measured by a selected high-throughput assay (such as cell growth or viability inhibition, induction of cell death or apoptosis, inhibition of cell migration, tumor growth inhibition, etc.) on each cells sets for each candidate compound combination, thereby defining a therapeutic window for each compound candidate compound combination;
**f)** Analysing the anti-cancer efficacy of each candidate compound in the defined therapeutic window and determining the contribution of each candidate compound as an individual agent and as a candidate compound pair to the overall activity of each of the candidate compound combinations generated under step d) by a second-order linear regression analysis;
**g)** Eliminating compounds from the candidate compound combinations based on a lack of synergistic interaction and/or no statistical significance in single compound first order terms in the second-order linear regression analysis model;
**h)** Reiterating steps d) to g) two to three by including in a new matrix under step d), candidate compounds at specified dosage levels and selecting under g) candidate compound combinations which show desirable compound interactions and result in sufficient inhibition of the targeted cell process selected in e).
**i)** Optimizing concentrations of compounds of the candidate compound combinations identified at the end of step h) after two to three iterations, by expanding the range of compound doses considered up to the maximum compound plasma concentration achievable in patients, including more concentrations values for each compound and exploring by experimental testing higher resolution design matrices and applying a regression model to identify the optimal compound concentration combination can be predicted as optimal solution of the regression model;
**j)** Identifying a low-dose candidate compound combination with the highest anti-cancer activity with minimal toxicity assessed under step g).

According to a particular embodiment, is provided a method for identifying low-dose multi-compound combinations, wherein clear cell renal cell carcinoma (ccRCC cell) (e.g. 786-O) are selected as malignant cells.

According to a particular embodiment, the candidates selected under step b) for the initial set of compounds are selected based on their stage of clinical evaluation (e.g. approved for clinical use in patient or have at least successfully complete phase I/II clinical trials) as well as their drug target. In particular, Compounds should be selected in a same set to target unique and complementary cell signalling pathways to increase the probability of identifying synergistic drug interactions.

According to another particular embodiment, the compound matrices are generated by combination of a two-level fractional factorial design with a three-level orthogonal array generating a resolution IV design matrix. Such matrices have previously been published in the literature (Xi et al., 2014, Phys Biol., 26, 11(6):065003. doi: 10.1088/1478-3975/11/6/065003) or can be developed *de novo* using appropriate statistical software (SAS Institute Inc., Statware Inc., SPSS Inc., etc.). Input requirements include the number of compounds to be screened (referred to as factors) and number of compound doses to be considered (referred to as levels, generally 3 in levels are applied in this methodology corresponding to two compound doses and a dosage of zero).

According to another particular embodiment, is provided a method for identifying low-dose multi-drug combinations, wherein human embryonic kidney 293 (HEK-293T) cells are selected as non-malignant cells.

According to another particular embodiment, is provided a method for identifying low-dose multi-drug combinations, wherein non-cancerous cells (cell lines) are from the same origin as the cancer of interest or other cells representing quantifiable indicators of toxicity, such as liver or renal function in animals, etc.

### Combined use and combined compositions

The invention provides pharmaceutical or therapeutic agents as compositions and methods useful for the treatment of cancer, in particular solid tumor cancers.
According to another aspect, the invention provides a use of a combination of CI-994 (PD-123654, GOE-5549, acetyldinaline), tubacin, erlotinib and dasatinib for the treatment of cancer, in particular of cancers associated with cancer cells forming multipolar spindles.
According to another aspect, the invention provides a use of a combination of CI-994 (PD-123654, GOE-5549, acetyldinaline), tubacin, erlotinib and dasatinib, applied in combination with anti-cancer drugs eliciting multipolar spindles, such as paclitaxel and epothilone B for the treatment of cancer.

CI-994, also called PD-123654, GOE-5549 or Tacedinaline or N-acetyldinaline or 4-(Acetylamino)-N-(2-aminophenyl)benzamide (CA registry n° 112522-64-2) has the following structure: 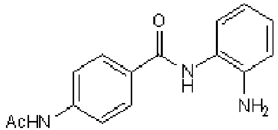 and is currently in phase III for the treatment of Non-Small Cell Lung Cancer (NSLC) (https://clinicaltrials.gov/ct2/show/NCT00005093) and was evaluated in phase I in advanced solid tumors (Pauer et al., 2004, Cancer Invest., 22(6):886-96)*.*
Tubacin, also called N-(4-{(2R,4R,6S)-4-{[(4,5-diphenyl-1,3-oxazol-2-yl)sulfanyl]methyl}-6-[4-(hydroxymethyl)phenyl]-1,3-dioxan-2-yl}phenyl)-N'-hydroxyoctanediamide (CAS registry n° 537049-40-4) the following structure: 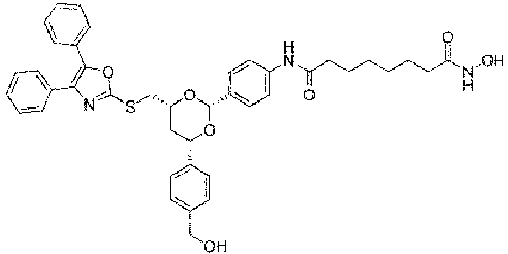 and is a tubulin acetylation inducer that selectively inhibits histone deacetylase (HDAC) 6 and is used as an experimental drug (Depetter et al., 2019, Int J Cancer., 2019, 145(3):735-747. doi: 10.1002/ijc.32169).
Erlotinib also called OSI-774, N-(3-Ethynylphenyl)-6,7-bis(2-methoxyethoxy)quinazolin-4-amine or 4-[(3-Ethynylphenyl)amino]-6,7-bis(2-methoxyethoxy)quinazoline (CAS registry n° 183321-74-6 and 183319-69-9 for the hydrochloride) has the following structure : 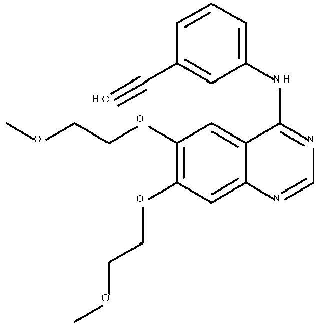 and is tyrosine kinase inhibitor, which acts on the epidermal growth factor receptor (EGFR) and Erlotinib hydrochloride (Tarceva™) has been authorized for the treatment of metastatic non-small cell lung cancer (*Rocha-Lima et al., 2009, 34(10):554-556, 559-564*) and in combination with gemcitabine for the treatment of metastatic pancreatic cancer (Moore et al., 2007, J Clin Oncol., 25(15):1960-6)
Dasatinib also called Sprycel™ (Bristol-Myers Squibb Company), BMS-354825 or N-(2-chloro-6-methylphenyl)-2-[[6-[4-(2-hydroxyethyl)piperazin-1-yl]-2-methylpyrimidin-4-yl]amino]-1,3-thiazole-5-carboxamide (CAS registry n° 302962-49-8) has the following structure 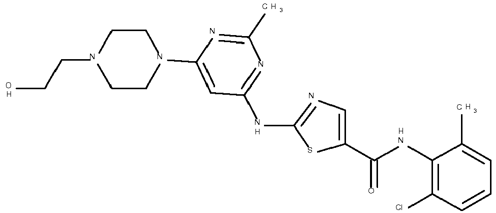 is a dual BCR/ABL and Src family tyrosine kinase inhibitor and has been authorized for the treatment of the treatment of paediatric patients with Philadelphia chromosome-positive (Ph+) chronic myeloid leukemia (CML) in the chronic phase (Gore et al., 2018, J Clin Oncol., 36(13):1330-1338. doi: 10.1200/JCO.2017.75.9597).
Paclitaxel (also called Taxol®, Onxal™, Clayman Chemicals) is classified as a "plan alkaloid," a "taxane" and an "antimicrotubule agent" (CAS registry n° 33069-62-4) has the following structure 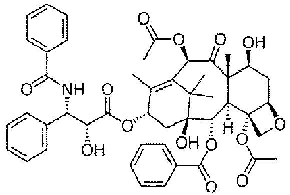 and has been authorized for the treatment of recurrent ovarian cancer (Menzin et al., Gynecol Oncol., 1994, 54(1):103), breast cancer, lung cancer, Kaposi sarcoma, cervical, bladder and pancreatic cancers (Drugs of choice for cancer. Treat Guidel Med Lett., 2003, 1(7):41-52).
Epothilone B is an antineoplastic agent and a microtubule-stabilising agent. It is an *epothilone* and an epoxide. Epothilone B is a 16-membered macrolide that mimics the biological effects of taxol (CAS registry n°152044-54-7). Epothilone B is an investigational drug, currently tested in clinical trials as anti-cancer drug against ovarian and breast cancers (Cheng et al., 2018, European Journal of Medicinal Chemistry, 157,925-934*)* which has the following structure. 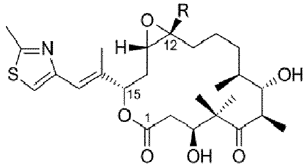 Its analog, Ixabepilone (Ixempra™, azaepothilone B, BMS-247550 was approved for the treatment of aggressive metastatic or locally advanced breast cancer.
According to a further aspect, the invention provides a combination of the following compounds: CI-994, tubacin, erlotinib and dasatinib for the treatment of cancer, in particular of cancers associated with cancer cells forming multipolar spindles wherein the said compounds are to be administered to an individual simultaneously or sequentially (e.g. multiple drug regimens). When said compounds or a pharmaceutical formulation thereof are administered simultaneously, those can be administered in the same or different composition(s) and by the same or different route(s) of administration. According to a particular aspect, using the same route of administration is preferable to more accurately account for drug dose variations arising from variable pharmacokinetics.
According to a further aspect, a combination according to the invention comprises CI-994 at a concentration of about 5 µM, tubacin at a concentration of about 5 µM, erlotinib (in particular erlotinib hydrochloride) at a concentration of about 5 µM and dasatinib at a concentration of about 6.3 µM.
According to another further aspect, a combination of the invention comprises 2.5 µM of CI-994, 5 µM of tubacin, 5 µM of erlotinib and 6.3 µM of dasatinib.

According to another further aspect, is provided a pharmaceutical composition comprising a combination according to the invention and at least one more pharmaceutically acceptable carrier.

According to a particular aspect, the combinations of the invention are to be administered through a single formulation.

Compositions of this invention may further comprise one or more pharmaceutically acceptable additional ingredient(s) such as alum, stabilizers, antimicrobial agents, buffers, colouring agents, flavouring agents, adjuvants, and the like.
The compositions according to the invention, together with a conventionally employed adjuvant, carrier, diluent or excipient may be placed into the form of pharmaceutical compositions and may be employed as solids, such as tablets or filled capsules, or liquids such as solutions, suspensions, ointments, emulsions, elixirs, or capsules filled with the same, films or gels, all for oral use. The compositions may also be formulated as a dry product for reconstitution with water or other suitable vehicle before use.
Compositions of this invention as liquid formulations including, but not limited to, aqueous or oily suspensions, solutions, emulsions, syrups, and elixirs.
Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.
Further materials as well as formulation processing techniques and the like are set out in
The Science and Practice of Pharmacy (Remington: The Science & Practice of Pharmacy), 22nd Edition, 2012, Lloyd, Ed. Allen, Pharmaceutical Press*, which is incorporated herein by* reference.
Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycolate. Wetting agents include, but are not limited to, sodium lauryl sulfate. Tablets may be coated according to methods well known in the art.

In a particular embodiment, the invention provides a pharmaceutical formulation according to the invention for use as a medicament.

### Mode of administration

The compounds of a combination of this invention may be administered independently any manner by oral route including to the mucosal surfaces of the oral cavity including the gingiva, the floor of the oral cavity, cheeks, lips, tongue, teeth, by intravenous or intramuscular or sub-cutaneous injection.

### Patients

In an embodiment, patients according to the invention are patients suffering from a cancer, in particular a solid tumor cancer.

In a particular embodiment, patients according to the invention are patients suffering from a cancer presenting a high percentage of cells prone to form multipolar spindles due to elevated centrosome number, centriole breakages, pre-mature centriole dis-engagement, or pericentriolar material fragmentation. Such cells are in particular frequently found in breast, ovaries, colon, brain, prostate, kidney, skin and lung cancers.

In another further embodiment, patients according to the invention are patients suffering from tumors of the skin, melanoma, lung, pancreas, breast, colon, rectum, brain, laryngeal, ovarian, prostate, colorectal, head, neck, testicular, lymphoid, marrow, bone, sarcoma, renal, sweat gland, and the like tissues.

In another further embodiment, patients according to the invention are patients suffering from breast and renal cancer.

### Use according to the invention

In a particular embodiment, the combined use of compounds of the invention and methods using this combination decrease or prevent the formation of centrosome clustering.

In another particular embodiment, the combined use of compounds of the invention and methods using this combination induce cancer cell apoptosis or senescence.

The method for identifying low-dose multi-drug combinations according to the invention advantageously allows identifying compound combinations which exhibit an increased specificity towards malignant cells due to the combination of the following constraints: (i) the parallel testing in malignant and non-malignant cells to create opportunities for combinations with a therapeutic window (TW) of activity and **(ii)** the inclusion of compound doses in the range of clinically attainable drug plasma concentrations allowing for efficient clinical translation (i.e. within the TW). Consequently, this resulted in the input compound dose of certain compounds in a compound candidate combination regression analysis under step f) that were even lower than the targeted ED₂₀ (e.g. axitinib, VX-680 and sorafenib) (Delaney et al., 2015, Oncotarget 6, 31104-31118, doi:10.18632/oncotarget.509).

Therefore, although counterintuitive to the optimization criteria which pushes to use the highest dose possible by screening compounds at doses where single compounds show no measurable effects, the method of the invention allows to take into account the heterogeneous treatment responses of different cell lines to clinically used drugs and by-pass the lack of efficacy of certain individual drug treatments at clinically relevant doses (Crusz et al., 2016, BMC medicine, 14, 185, doi:10.1186/s12916-016-0729-9) for successfully identifying optimized compound combinations. In fact, modeling analysis of both efficacy (786-O cell viability inhibition) and the TW (difference between non-malignant and malignant cells) led to the identification of a 786-O cell-specific and synergistic optimal drug combination composed of CI-994, tubacin, erlotinib and dasatinib (drug combination C2) which exhibits superior activity as compared to the current first line standard ccRCC treatment (sunitinib).

Therefore, in contrast to other approaches based on pharmacogenetics or high-throughput screening for drug combinations, the method of the invention allows to identify optimized, synergistic low-dose multi-drug combinations with minimal experimental effort.
According to another aspect, the method according to the invention does not require background and mechanistic information about the system prior to the start of optimization. According to another aspect, the method according to the invention advantageously allows to identify low-dose compound combinations (below the clinical plasma concentrations) which are inactive in normal cells therefore reducing side effects.
The cellular assays used for determining the activities under steps c) and/or d) might be performed using automated screening platforms/robots for multiple agent testing. In particular, the compound combinations can be automatically prepared and distributed to cells in multi-well plates. Assay readouts can also be automated used plate readers and robotic pipetting of reagents.

The method of the invention which includes in the screening (i) compound combination optimization in the non-cancerous cells simultaneously to optimization in the cancerous cells and (ii) compound combination optimization based clinically-relevant compound doses advantageously drives the selection of non-toxic compound combinations that can be further developed at the clinically relevant concentrations.

This represents a major non-obvious advantage over the existing methods for identification of drug combination which do not take toxicity into consideration, thus tending towards the identification of combinations containing more compounds at higher doses, increasing the probability of identifying combinations with increased toxicity. Furthermore, these known methods employ a population-based search algorithm in order to identify drug combinations with enhanced efficacy. As such, those methods (e.g. WO 2015/136061) require the evaluation of a greater number of drug combinations as well as more than ten iterative search rounds, slowing the speed and cost of optimization searches.

Examples illustrating the invention will be described hereinafter in a more detailed manner and by reference to the embodiments represented in the Figures.

### EXAMPLES

The following abbreviations refer respectively to the definitions below:
**DMEM** (Dulbecco/Vogt modified Eagle's minimal essential medium); **DMSO** (Dimethyl Sulfoxyde); **RPMI 1640** (Roswell Park Memorial Institute).

### Example 1: Method of identifying drug combinations according to the invention

The method of the invention is based on Therapeutically Guided Multidrug Optimization (TGMO)-based optimization of drug combination. The method comprises the following steps as schematized under Figure 1A:

### a) providing separately a set of human clear cell renal cell carcinoma (ccRCC) 786-O cell line as cancer cells (malignant) and a set of non-malignant human embryonic kidney 293 (HEK-293T) cells as non-cancer cells (non-malignant)

ccRCC were chosen as malignant cells because those are the most aggressive and common type of kidney cancer, representing about 70% of kidney tumors *(*Baldewijns et al., 2008, Biochim Biophys Acta, 1785, 133-155, doi:10.1016/j.bbcan.2007.12.002). The selection of this type of cells was also dictated by the chemo-and radio-resistant properties of ccRCC.

786-O cells were selected based on their lack of sensitivity to HDACI and known resistance to sunitinib, the first line of treatment for ccRCC. Culture media consisted of RPMI for 786-O and HEK-293T cells. All cells were recently been tested for mycoplasma contamination and been authenticated and by the PCR method.

### b) selecting an initial set of candidate compounds to be assayed

Compounds should be selected based on their stage of clinical evaluation as well as their drug target. Compounds should be approved for clinical use in patient or have at least successfully complete phase I/II clinical trials. Compounds should be selected to target unique and complementary cell signalling pathways to increase the probability of identifying synergistic drug interactions.

A set of 10 candidate compounds was selected compounds that belong to tyrosine kinase inhibitors (TKI), histone deacetylase inhibitors (HDACI) and pan-Aurora kinases inhibitors as follows:
This selection was based on successful pharmacological intervention with kinase inhibitors (KI) and histone-deacetylase inhibitors (HDACI) in the clinic. Moreover, many HDACI sensitize cancer cells to both cytotoxic and targeted agents (Marcus et al., 2005, Cancer Res 65, 3883-3893, doi:10.1158/0008-5472.CAN-04-3757) and various clinical and preclinical data suggests synergistic interactions between KI and HDACI (LaBonte et al., 2017, Cancer research, 71, 3635-3648, doi:10.1158/0008-5472.CAN-10-2430*;* Chen et al., 2013, Cell death & disease, 4, e810, doi:10.1038/cddis.2013.330*;* Thurn et al., 2011, Future oncology 7, 263-283, doi:10.2217/fon.11.2*;* Berndsen et al., 2017, Angiogenesis, 20, 245-267, doi:10.1007/s10456-017-9551-z).
- Six **kinase inhibitors** were included: **axitinib, erlotinib, BEZ-235, dasatinib, VX-680 (Tozasertib) and sorafenib. Axitinib** (Inlyta®) is a second generation TKI targeting selectively (VEGFR) 1, 2 and 3, and at lower affinity PDGFR-B and c-KIT and is clinically used in the treatment of advanced RCC Bellesoeur et al., 2017, Drug design, development and therapy 11:2801-2811*;* Keating, 2015, drugs, 75(16):1903-1913). **Erlotinib** (Tarceva®) is used for treatment of non-small cell lung carcinoma to limit use to patients whose tumors have specific epidermal growth factor receptor (EGFR) mutations and for the treatment of patients with locally advanced, unresectable or metastatic pancreatic cancer, in combination with gemcitabine. It has also been tested in a treatment of advanced papillary RCC in combination with bevacizumab (Modi et al., 2016, Ann. Transl. Med. 4(7):143). **BEZ-235** (DactolisibO) is imidazoquinoline derivative acting as a PI3K and mTOR inhibitor that induces tumor cell apoptosis and growth inhibition in PI3K/mTOR-overexpressing tumor cells. It was tested in advanced RCC as monotherapy in phase1b clinical trial that was terminated due to toxicity constrains (Carlo et al., 2016, Oncologist, 21(7):787-788). **Dasatinib,** a multi-kinase inhibitor which was originally approved for the treatment of Imatinib-Resistant Philadelphia Chromosome-Positive Leukemias as a result of its activity against the BCR-ABL tyrosine kinase. It has also been shown to act on the Src-YAP signalling axes, which would represent its major target in the treatment of solid tumors, including RCC *(*Araujo et al., 2010, Cancer Treat Rev 36(6):492-500*).* **VX-680** (Tozasertib®) selectively targets the Aurora-A,-B,-C kinases, which is involved in cell cycle choromose segregation and cytokinesis (Harrington et al., 2004, Nat Med, 10(3):262-267). Inhibition of this kinase has been shown to induce cell cycle arrest and apoptosis (*Harrington et al., 2004, supra)* and it has undergone investigated in clinical trials for various solid tumors *(*Mountzios et al., 2008, Cancer Treat Rev 34(2):175-182*).* **Sorafenib** is a clinically approved multi-target TKI inhibiting various kinases, mainly Raf-1, B-Raf and VEGFR-2. Sorafenib has shown clinical efficacy in the treatment of advanced and metastatic ccRCC (Ratain, et al., 2006, J Clin Oncol 24(16):2505-2512*;* Escudier et al., 2007, N Engl J Med 356(2):125-134), however with relatively high toxicity due to off-target effects.
- Four histone deacetylase inhibitors are included in this screen, **CI-994, LBH-589, SAHA and tubacin. CI-994** targets class I HDACs. It has been investigated in clinical trials in combination with various chemotherapeutic agents for the treatment of solid cancer with varying success (Pauer et al., 2004, Cancer Invest 22(6):886-896*;* Richards et al., 2006, Ann Oncol 17(7):1096-1102). **LBH-589** targets Class I and II HDACs and has obtained FDA approval for the treatment of multiple myeloma in combination with bortezomib and dexamethasone after at least two other prior treatment regimens (San-Miguel et al., 2014, Lancet Oncol, 15(11):1195-1206). It has also been clinically examined for the treatment of RCC and other solid tumors alone (Hainsworth et al., 2011, Cancer Invest 29(7):451-455) and in combination with bevacizumab and everolimus (Strickler et al., 2012, Cancer Chemother. Pharmacol. 70(2):251-258*).* **SAHA** is also a Class I and II HDAC inhibitor approved for advanced primary cutaneous T-cell lymphoma (Mann et al., 2007, Oncologist 12(10):1247-1252). It is also being clinically evaluated in various combination therapies for solid tumors, including with sorafenib (Dasari et al., 2013, Invest New Drugs, 31(1):115-125). Finally, **tubacin** is a Class IIb HDAC inhibitor selective targeting HDAC 6 (Tandon et al., 2016, Clin Pharmacol., 8:35-44*).* HDAC6 has been identified to play a role in the malignant progression of cancer has been identified as a relevant therapeutic target (Aldana-Masangkay et al., 2011, J. Biomed. Biotechnol., 2011:875824).

CI-994, LBH-589, SAHA, axitinib, erlotinib HCl, BEZ-235, dasatinib, and sorafenib were purchased from LC Labs (Woburn, MA, USA). Tubacin (SML0065) was purchased from Sigma-Aldrich (St. Louis, MO, USA) and VX-680 from Selleck Chemicals (Houston Texas, USA). Compounds were dissolved in sterile DMSO (Sigma-Aldrich) at the following concentrations: CI-994 (20 mg/ml), LBH-589 (5 mg/ml), SAHA (5 mg/mL), tubacin (5 mg/ml), axitinib (20 mg/ml), erlotinib HCl (15 mg/ml), BEZ-235 (1 mg/ml), dasatinib (5 mg/ml), VX-680 (20 mg/ml) and sorafenib (40 mg/ml). Aliquots were stored at -80°C and thawed prior to each experiment. A maximal concentration of 0.1% DMSO was allowed for any of the screened conditions and was used as a control (CTRL).

### c) contacting the said set of cancer cells and set of non-cancer cells with each of the selected candidate compound and measuring metabolic activity of said cells for different candidate compound concentrations, thereby generating dose-response curves for each candidate compound (Figure 2)

The experiments were performed in a simple *in vitro* cell viability bioassay measuring cell metabolic activity and using inhibition of metabolic activity as a measure for cell viability wherein cells were seeded in 96-well culture plates and 24h post-seeding 72h treatments with 0.1% DMSO CTRL or drug (combinations) were initiated. Seeding density was 2-10 x 10³cells/well, depending on the cell line's growth characteristics such that the control cells approach confluency at the time of the assay readout. Subsequently, cell viability was assessed using the CellTiter-Glo® luminescence assay (Promega, Madison, WI, USA) and cell viability was reported as the luminescence signal in the treated wells normalized to the signal in the DMSO treated control wells. Dose-response curves and IC₅₀ values were determined in Graphpad Prism® using a four-parameter non-linear fit of the log transformed dose data of each compound. The ED₅₀ dose (dose that inhibited 50% of cell viability, as compared to the control) of each candidate compound was determined by fitting dose response data on a semi-log scale. The ED₂₀ (or lower, based on clinically attainable drug plasma concentrations based on published data from phase I/II clinical trials as the average blood plasma dose obtained in patients over a 24 hour period at a tolerated drug dose) was selected as the highest dose for combinatorial drug screening **(Table 1).**

**Table 1**

| **Drug** | **786-O** | | **Target** |
|---|---|---|---|
| | **ED₅₀ (µM)** | **ED₂₀ (µM)** | |
| CI-994 | 11.5 | 1 | Class I HDACs |
| LBH-589 | 0.01 | 0.002 | Class I and II HDACs |
| SAHA | 3.7 | 1 | Class I and II HDACs |
| tubacin | 6 | 2 | HDAC6 |
| axitinib | 11.5 | 0.2 | VEGFRs, PDGFR |
| erlotinib | 6.6 | 2 | EGFR |
| BEZ-235 | 0.09 | 0.02 | mTOR |
| dasatinib | 0.1 | 0.05 | SRC |
| VX-680 | 33 | 0.65 | pan-Aurora Kinases |
| sorafenib | 2.1 | 0.01 | VEGFRs, PDGFR |

### d) designing a compound candidate combination matrix by orthogonal array composite design (OACD) and selecting a set of candidate combinations(Figure 1A, II).

Candidate combinations for further experimental testing were defined by an orthogonal array composite design (OACD) (Xu et al, 2014, Statistica Sinica, 24:269-289) which allows to define a set of experimental data points (i.e. compounds and corresponding compounds doses) to be tested. Compound matrices are generated by combination of a two-level fractional factorial design with a three-level orthogonal array generating a resolution IV design matrix. Such matrices have previously been published in the literature (Xi et al., 2014, Phys Biol., 26, 11(6):065003. doi: 10.1088/1478-3975/11/6/065003) or can be developed de novo using appropriate statistical software (SAS Institute Inc., Statware Inc., SPSS Inc., etc.). Input requirements include the number of compounds to be screened (referred to as factors) and number of compound doses to be considered (referred to as levels, generally 3 in levels are applied in this methodology corresponding to two compound doses and a dosage of zero).The two-level fraction factorial design allows for the estimation of linear and bi-linear effects (i.e. single compound and 2-compound interaction coefficients, while the three-level orthogonal design allows for the estimation of linear and quadratic effects. Thus, the design allows for internal cross-validation of linear effects. The resulting design is a resolution IV matrix and allows for accurate screening for the most influential factors in the system based on accurate estimations of each factor's main effect (i.e. main effects are not aliased by other main effects or two-factor effects), in addition to estimates of interaction and quadratic effects. 91 compound combinations were generated according to the drug combination matrix (IV). Those drug combinations contain from zero to 10 compounds in combination and defining both the compounds and the dosage (ED₂₀ or half ED₂₀) to be screened in the next steps.

### e) contacting a new set of cancer cells and a new set of non-cancer cells with each of the candidate compound combinations designed under step d) and measuring the difference in efficacy of cell metabolic activity on each cells sets for each candidate compound combination, thereby defining a therapeutic window for each candidate compound combination (Figure 1A, III).

The generated combinations were they assayed for their efficacy on both types of cells (malignant and non-malignant) measured in *in vitro* cell viability inhibition assays (cell metabolic activity) and the difference in compound mixture efficacy between non-malignant HEK-293T cells and malignant 786-O cells was defined as the therapeutic window (TW).

### f) Analysing the efficacy of each candidate compound in the defined therapeutic window and determining the contribution of each candidate compound as an individual agent and as a candidate compound pair to the overall activity of each of the candidate compound combinations generated under step d)by a second-order linear regression analysis (Figure 1A, IV, Figure 3)

The efficacy values of the cell metabolic activity inhibition in the therapeutic window were used to generate second-order linear regression models (Matlab®). For each regression model, an estimated regression coefficient was generated for the significant terms in the model, including "single-drug first order", "two-drug interaction", and "single-drug second-order". These terms describe the contribution of each candidate compound as an individual agent and as a candidate compound pair to the overall activity of the candidate compound combinations. The accuracy and reliability of the regression models was assessed based on residual analysis **(****Figure 3b****).** Finally, an ANOVA lack of fit test was performed to confirm the selection of an appropriate model structure. Higher order terms, such as three compound interactions, are generally considered to make a negligible contribution to overall activity and are therefore not included in the analysis (Wood et al., 2012, Proc Natl Acad Sci USA, 109, 12254-12259*, doi:10.1073*/*pnas.1201281109).*

Regression models were used to eliminate compounds showing poor single drug activity or antagonistic drug interactions from the data set or those that do not have synergistic interactions with other drugs from the set subjected to the subsequent analysis round (reiteration of steps d) to g), thereby a smaller subset of candidate compound is subjected to the further OACD design and analysis based on regression modelling. The generated models made it possible to analyse compound-compound interactions and to identify synergistic, selective compound interactions, guiding further compound selection and elimination. This facilitated refinement of the compound candidate combinations to be tested in the subsequent experimental rounds and the selection of compound combination with maximal cancer cell viability inhibition and minimal toxicity in non-cancerous cells (**Figure 1A****, V)** as follows: First two frames from the left indicate candidate compounds showing not optimal compound interactions (either high efficacy with low toxicity or low efficacy with no toxicity), while candidate compounds exhibiting an optimal activity profile, being both effective and non-toxic, are framed in dark grey. The black frame indicates candidate compounds with poor activity profiles (lack of efficacy and considerable toxicity).

Therefore, in the next round of analysis, only combinations presenting optimal activity profile, i.e. being both effective and non-toxic are selected and further subjected to a second round of analysis, as determined based on regression models of describing the activity and therapeutic window of combinations. Three sequential rounds of testing (steps d) to g)) using OACD matrices and regression analysis of such analyses allow the identification of compound combinations with beneficial synergistic activity, namely maximal cancer cell viability inhibition and minimal toxicity in non-cancerous cells.

### h) Reiterating steps d) to g) by including in a new matrix under step d) candidate compounds which show strong single drug activity or synergistic drug interactions and selecting under g) candidate compound combinations which are effective and lack toxicity under step e)

Compound doses used in compound combinations was half the ED₂₀. Example of iterative analyses are shown under **Figure 1 B-E****:**
**Step f) of the first round analysis (fl):** the 91 compound combinations were screened and stepwise regression analysis of the experimental data led to the elimination of three compounds, i.e. LBH-589, SAHA and axitinib, due to the lack of synergistic interaction and/or no statistical significance in single compound first order terms **(****Figure 1B**).

Then, two combination sets were assayed as follows:
- A set of 50 combinations composed of up to 7-compounds (combinations with 2, 3, 4, 5, 6 or 7 compounds in the same set) taken from the initial compound set without the excluded three compounds **(****Figure 1C and D****);**
- a set of 25 combinations composed of 4 compounds (combinations with 2, 3 or 4 compounds in the same set) taken from the above compound set without the excluded three compounds **(****Figure 1E**).

Each of those two combination sets were defined by applying a new OACD design step d) (d2 and d3). The 4-compound combination set comprised CI-994, tubacin, erlotinib and dasatinib and was selected under a step **gl)** based on the outcome of **step f1)** on the above set of 50 combinations and.

The parallel analysis based on regression modelling of the 7-compound combination set led to the identification of promising 4-compound combinations **(****Figure 1E**). The synergy between tubacin and erlotinib was confirmed in all three of the 4-compound sets investigated. The two 4-compound sets investigated in steps **e3)** to **g3)** did not show improved efficacy over the original 4-compound combination set screened in steps **e2)** to **g2),** as synergy was driven by the same interaction between tubacin and erlotinib and first order effects were not superior to the primary selection. Thus, the method of the invention identified one selective four drug combination (**C1**), consisting of CI-994, tubacin, erlotinib and dasatinib.

The efficacy of **C1** was driven by a strong synergy between tubacin and erlotinib, as identified in the second and third iteration steps of the analysis based on regression modelling according (steps e2 and e3) and f2) and f3) **(****Figures 1B-E****,** highlighted in grey, as well as by the strong activity of erlotinib and dasatinib. The activity of **C1** showed highly selective and synergistic activity, as indicated by **C1** outperforming the corresponding monotherapies and by the lack of activity in the non-malignant HEK-293T cell line **(****Figure 4A****).** Response surfaces generated from the regression model of data obtained in the second iteration step of the analysis based on regression modelling according (steps e2) and f2) **(****Figure 1E**), demonstrated the synergistic interaction of tubacin and erlotinib (as evidenced by the slope of the surface), as well as the important contribution of all four compounds in the optimized combination **(****Figure 4B****).**

### i) Optimizing concentrations of compounds of the candidate compound combinations identified at the end of step h) after the three iterations d) to g)

Finally, a broader dose range of these four compounds was considered and doses of compounds included in **C1** were optimized as follows. 786-O cell viability inhibition after incubation with combinations **C2-C5** and corresponding monotherapies are presented in **Figure 5A and B****. C2** inhibited 786-O cell viability by 96%, showing a significantly greater activity than C1 (p<0.0001) and all single compound treatments. Drug combinations **C1-C5** were only minimally active in HEK-293T, as well as normal human fibroblast NHDFa cells, confirming the successful application of the therapeutic window-based drug optimization according to the method of the invention. Moreover, **C1-C5** also significantly outperformed the activity of non-optimal random drug combinations **(****Figure 4****),** validating the selection resulting from a method of the invention. The synergistic potential of each of the combinations was further analysed by calculating their respective Combination Indexes (CI) using Compusyn© software, based on Loewe additivity (Fouquier et al., 2015, Pharmacol Res Perspect., 3(3): e00149; Ting-Chao Chou, 2010, DOI: 10.1158/0008-5472.CAN-09-1947). While CI values lower than 1 signify synergistic drug combinations (highlighted in gray), CI higher than 1 indicate antagonism and a CI between these values indicates additivity **(****Figure 5A****).**

### j) Identifying a low-dose candidate compound combinations with the best activity (cell metabolic activity inhibition vs. control)

**C2** (2.5 µM of CI-994, 5 µM of tubacin, 5 µM of erlotinib and 6.3 µM of dasatinib) showed over 10-fold higher synergy (CI=0.04) than other combinations and was hence selected for further evaluation.

Therefore, starting from a set of only four HDACI and six KI, the method of the invention allowed to powerfully identify a selective drug combinations after testing only 0.37% of all possible experimental conditions accompanied by modelling-based data analysis.

### Example 2: Confirming the efficacy of a drug combination according to the invention

The activity of the identified optimized drug combination **(C2)** was tested in cell viability inhibition was further tested in 3D homotypic (786-O cells) and 3D heterotypic (composed of 786-O cells, complemented with normal human dermal fibroblast adult (NHDFa) fibroblasts (Lonza) in ratio 1:1 and 10% human endothelial cells ECRF24) cell culture models as follows:

### Three-dimensional cell cultures

The efficacy of **C2** and corresponding monotherapies was evaluated in homotypic 786-O, seeded 3000 cells/well, and heterotypic three-dimensional (3D) models. The latter model consisted of 3000 786-O cells co-cultured with 3000 NHDFa cells (DMEM medium) and 600 cells of freshly Human umbilical vein endothelial cells (HUVEC) isolated as indicated below (corresponding to 10%). Cell mixture was supplemented with 2,5% matrigel (Corning matrigel, #354230) and 50 µl/well was seeded in round bottom 96-well plate with cell-repellent surface (Cellstar® 650970) by using an electronic multi-dispensing pipette at low speed. The plate was centrifuged at 400 x g at 4°C. The whole procedure was performed on ice and materials were pre-cooled to avoid polymerization of matrigel. The co-cultured spheroids were incubated with drugs for 72 hours. Cell viability was measured with CellTiter-Glo® 3D Cell Viability Assay (Promega, #G9681). Signal was measured with a luminescent plate reader (Biotek citation 3 with corresponding software at the standard settings).

HUVEC were isolated from human umbilical cords and used for experiments only until passage 4. HUVEC cells were maintained in complete M199 medium (Gibco by ThermoFischer Scientific) prepared freshly every week, supplemented with 10% Fetal Bovine Serum (FBS), 1% penicillin/streptomycin (ThermoFischer Scientific), 1% Endothelial Cell Growth supplement (ECGS; Millipore), 0.1 mg/mL heparin sodium salt (Sigma-Aldrich), 0.1 µM Hydrocortisone (Sigma-Aldrich) and 10 µg/mL L-ascorbic acid (Stock 10 mg/ml, dilute 1:1000, Sigma-Aldrich). HUVECs were grown in cell culture flasks coated with 0.2% gelatin (Sigma-Aldrich) and 1 mg/mL Collagen G (Biochrom AG) in PBS.

As seen on **Figures 5C****, C2** induced an effective, approx. 80% cell viability inhibition in those models (p<0.001 vs. CTRL and all monotherapies), confirming the results obtained in the 2D cell cultures as described above in Example 1 **(****Figure 5A****).**

### Example 3: Anti-angiogenic efficacy of a drug combination according to the invention

Since anti-angiogenic treatment may potentiate the overall treatment of ccRCC, human immortalized ECRF24 endothelial cells macrovascular human endothelial cells immortalized (via immortalization procedures with amphotrophic replication-deficient retrovirus as described in Fontijn et al., 1995, Exp Cell Res., 216(1):199-207) were exposed to C1-C5 and corresponding monotherapies.

### Two-dimensional cell migration assay

The 2-dimantional cell migration assay was performed in a endothelial cell scratch assay as described in van Beijnum et al., 2017, Angiogenesis, 10.1007/s10456-017-9576-3, doi:10.1007/s10456-017-9576-3*.* 786-O cells were seeded at a density of 3-3.5 x 10⁴ cells/well) cells in a 96-well cell culture plate and grown overnight. A scratch was made using a sterile scratch tool (Peira Scientific Instruments, Beerse, Belgium) and the treatment was administered immediately after. The scratch wounds were imaged using a Leica DMI3000 microscope (Leica, Rijswijk, Netherlands) at x5 magnification using Universal Grab 6.3 software (DCILabs, Keerbergen, Belgium). Imaging of was performed at T = 0h and several time points after scratching until T = 7h. The size of the scratch was automatically quantified and analysed using Scratch Assay 6.2 (DCILabs) by calculating the absolute wound closure (initial minus final scratch surface) as described in van Beijnum et al., 2013, Oncogene, 17, 363-374*.*

Interestingly, while **C2** and **C3** were virtually inactive in NHDFa cells, they were quite potent in activated human endothelial cells ECRF24 cells. These drug combinations induced inhibition (66% and 52%, respectively) of ECRF24 cell viability. Treatment with **C2** also led to increased apoptosis induction in endothelial cells (12.6% vs. 1.6% in CTRL, **Figure 6A****),** which was mainly driven by tubacin, but did not influence endothelial cell migration in 2D **(****Figure 6B****)** or 3D network formation **(****Figure 6C****).** The anti-angiogenic potential of **C2** was further confirmed in the *in vivo* developmental chorioallantoic membrane (CAM) model as described in Nowak-Sliwinska et al., 2018, Angiogenesis, 21, 425-532, doi:10.1007/s10456-018-9613-x and Nowak-Sliwinska et al., 2014, Angiogenesis, 17, 779-804, doi: DOI 10.1007/s10456-014-9440-7*,* where **C2** significantly (p<0.0001) reduced the development of the microvasculature (quantified by the number of branching points/mm2, **Figure 6D****).** These results suggest therefore a potential dual, i.e. anti-cancer and angiostatic, mechanism of action of **C2.**

### Example 4: Activity of a drug combination according to the invention activity on a cell cycle regulation, actin cytoskeleton reorganization and nuclear structures

Cell cycle distribution and the frequency of apoptosis were monitored by flow cytometry after propidium iodide staining and assessment by flow cytometry.

Exposure of 786-O cells to **C2** for 24 hours resulted in higher apoptosis levels (8.1% vs. 1.1% in CTRL, p=0.0109). After 72 hours of drug incubation, the higher incidence of apoptosis was no longer observed, however, a G2/M phase arrest was registered (12.3% and 21.3% for CTRL and C2, respectively, p=0.0083, Supplementary Figure S6a bottom graph). Sunitinib at a concentration of 10 µM was used as positive control.

Morphological changes in **C2**-treated cells were also investigated based on fluorescent phalloidin (f-actin marker) and nuclear DAPI staining. 786-O cells were seeded on glass in a 24-well plate with a density of 6000 cells/well. After 72 hours of treatment with optimized drug combination or monotherapies the cells were fixed with 4% formaldehyde for 10 minutes at RT, washed twice and permeabilized with 0.1% Triton-X for 15 minutes. Cells were incubated at RT with Alexa Fluor 488 Phalloidin (A12379, Thermo Fisher) diluted 1:200 for 20 minutes to stain f-actin, washed twice and incubated at RT with 1 ug/mL Dapi (D9542, Sigma) for 5 minutes. After a final wash-step the glasses were imaged on the Biotek Cytation3 Imaging reader using a 10x objective.

After C2 treatment, changes in the actin cytoskeleton morphology were observed. The cells presented enhanced stress fibers in the center, but also the formation of f-actin cables running along the cell borders. Nevertheless, C2 did not affect 786-O migration in a 2D migration assay **(****Figure 6b****).** DAPI staining also indicated that **C2** treatment led to a 3-fold increase (7.5% ± 1.3%) in the number of micronuclei vs. CTRL treatment (1.8 ± 0.3%, p<0.001, **(****Figure 6b****).** Micronuclei induction is a marker for chromosome segregation errors or chromosome aberrations reported as being linked to genomic instability and chromosomal damage (Leibowitz et al., 2015, Annual review of genetics, 49, 183-211, doi:10.1146/annurev-genet-120213-092228*).*

### Example 5: Activity of a drug combination according to the invention activity on cancer cell division

In order to test whether **C2** treatment affects cell division, this process was monitored by live cell imaging. 786-O cells were treated with 0.1% DMSO in the cell culture medium (CTRL) or **C2** and stained with the live cell dye SiR-tubulin (microtubule marker) to label the mitotic spindle, monitor mitotic progression and detect any cytokinesis failure. All experiments were performed in the presence of Valspodar (4 µM), a multi-drug pump inhibitor that prevented SiR-tubulin removal from the cell (this concentration of Valspodar had no effect on the efficacy of **C2).** Live cells were recorded for 24 hours at a temporal resolution of 3 minutes **(****Figure 7A****).**

Those experiments revealed that unexpectedly, **C2**-treated cells spent a longer time in mitosis as the mean time between nuclear envelope breakdown (NEBD, time point at which the spindle is set up) and the appearance of the cleavage furrow, 30 ± 10 min in CTRL-treated cells and 42 ± 6 min in **C2-**treated cells **Figure 7A& B****).** A number of **C2**-treated cells died during interphase (6% vs. 1%), and fewer cells entered mitosis (35% vs. 66%), pointing to a delay in the cell cycle **(****Figure 7C****).** Among the cells entering mitosis, fewer cells completed mitosis and survived, and more cells died, either during mitosis (3% vs. 0%) or after mitotic exit in the ensuing interphase (12% vs. 0.5%, **Figure 7D****).** Even though treating the cells with erlotinib or tubacin alone induced a small delay in mitosis, no phenotype as striking as the ones observed with **C2** were noted. There was no evidence that cells could not perform cytokinesis. The most striking phenotype, however, was the frequent presence of multipolar spindles in **C2**-treated cells (44% vs. 18% in CTRL-treated cells, **Figure 7E****).**

### Centrosome staining

Cells were seeded on glass in a 6-well plate with a density of 100 000 cells/well for 786-O, 786-OsunR and MDA435 150 000 cells/well for M14 cells the day prior to the experiment. Cells were fixed with methanol at -20°C for 6min, rinsed with PBS, blocked with 3% BSA RT, then stained for 1 hour RT. The following primary antibodies were used: rabbit serum anti- ytubulin (1:2000, this study) and mouse anti-centrin (1:2000, Merck Millipore, clone 20H5). Cells were incubated at RT with Alexa Fluor 488 anti-mouse (A21202, Invitrogen) and Alexa Fluor 594 anti-rabbit (A11012, Invitrogen) diluted 1:400 for 30 minutes. Coverslips were mounted with VECTASHIELD with DAPI (Vector Laboratories). Z-stack images were taken using an Olympus DeltaVision wide-field microscope (GE Healthcare) with 60x 1.4 NA oil objective and recorded with a Coolsnap HQ2 CCD camera (Roper Scientific) and the Softworx software (GE Healthcare) with 0.2 µm spacing between stacks.

### Mitosis live cell imaging

To visually monitor the effect of the drug combination **C2** on 786-O cells, live cell imaging was performed for 24 h at 37°C on the Ti widefield microscope (Nikon) equipped with an environmental chamber using a 60× 1.3 NA oil objective and a CoolSNAP HQ camera (Roper Scientific) at a sampling rate of 3 min, recording at each time point of 9 Z-stacks separated by 2 µm. 20 points were taken per condition. 2 hours before the start of the movie, SiR-Tubulin was added on the cells (SpiroChrome, 50 nM) to stain microtubules, with Valspodar (4 µM) to keep the dye inside the cells. Time-lapse movies were analysed using NIS Elements AR Software and cropped into figures using FIJI software (ImageJ; National Institutes of Health) and Adobe Illustrator CC (Adobe).

In mammalian cells, centrosomes represent the major microtubule organizing centers and are responsible for the formation of a bipolar spindle during mitosis (*Fu et al., 2015, Cold Spring Harb Perspect Biol, 7, a015800, doi:10.1101*/*cshperspect.a015800*). Having more than two centrosomes contribute to the production of multipolar cell division that in turn frequently leads to cell death in the ensuing interphase due to massive chromosome mis-segregation *(*Nigg, 2002, Nat Rev Cancer, 2, 815-825, doi:10.1038/nrc924*;* Godinho et al., 2014, Biological sciences, 369, doi:10.1098/rstb.2013.0467). Cancer cells possess a survival mechanism to overcome this problem called centrosome clustering (CC), where supplementary centrosomes or spindle poles are gathered together to form a pseudo-bipolar spindle, a phenomenon that was also visible in 786-O cells **(****Figure 7A****,** white arrows). Therefore, the percentage of multipolar spindles was plotted over time and found that **C2-**treated cells were severely delayed in their ability to cluster multipolar spindles compared to the CTRL, resulting in 15% of the cells failing to resolve their multipolar spindles over the entire course of mitosis **(****Figure 7A and F****).** This phenotype seems to be due specifically to drugs synergy, as none of the monotherapies could induce an inhibition of the clustering by themselves.

### Example 6: Activity of a drug combination according to the invention activity on spindle pole clustering in sunitinib-resistant 786-O cells

The first-line therapy for advanced ccRCC is sunitinib, a tyrosine kinase inhibitor to which patients rapidly develop resistance. Therefore, it was evaluated whether drug combination of the invention **C2** would also be effective in sunitinib-resistant cells (786-OsunR) by live cell-imaging experiments after CTRL- or **C2**-treatment.

First, 786-O cells were chronically exposed to sunitinib treatment (1 µM) and dose response curves were generated to validate resistance induction. Moreover, intracellular and intralysosomal accumulation, one of the resistance mechanism of sunitinib (Adar et al, 2012, Cell Death Disease, 3, 1-10, doi: 10.1038/cddis.2012.30) was observed. **C2** effectively inhibited the viability of 786-OsunR cells and significantly outperformed all single drug treatments **(****Figure 8A****,** p=0.0001 vs. CTRL and all monotherapies). While **C2** barely affected mitotic timing **(****Figure 8B****),** it prevented spindle pole clustering into a bipolar spindle over the course of mitosis by 30% in 786-OsunR cells (vs. 5% for CTRL; **Figure 8C** and **D****).** This proved that **C2** prevented bipolar spindle formation in a large segment of 786-O cells independently of sunitinib-induced resistance (Nigg et al., 2011, Nature cell biology, 13, 1154-1160, doi:10.1038/ncb2345)*.* In contrast to non-malignant cells that possess 2 centrosomes with 2 centrioles, cancer cells frequently have an elevated number of centrioles/centrosomes, making it the primary cause for multipolar spindles (Zyss et al., Trends Cell Biol, 19, 334-346, doi:10.1016/j.tcb.2009.04.001).

786-O and 786-OsunR cells, however, display only a moderate percentage of cells with elevated centriole numbers, consistent with previous studies (Marteil et al., 2018, Nat Commun, 9, 1258, doi:10.1038/s41467-018-03641-x*)* **(****Figure 8E** and **F****).** This indicated that **C2** can prevent spindle pole clustering independently of abnormal centrosome numbers. Concurrently, **C2**-treatment also led to a high proportion of cell death in interphase, before the **C2** treated-cells could enter mitosis, raising the hypothesis that **C2** could have multiple mechanisms of action **(****Figure 8G****).** Focusing on cells entering mitosis **(****Figure 8H****),** less cells survived when treated with **C2** (32% vs. 93% in CTRL), and more cells died either during (17% vs. 1% in CTRL) or after mitotic exit (41% vs. 2% in CTRL).

### Example 7: Differential activity of a drug combination according to the invention on cancerous versus non-cancerous kidney cells

The main advantage of **C2** is its efficacy on ccRCC cells compared to HEK-293T cells **(****Figure 5A****).** Live cell imaging performed on these control cells showed that even though C2 increases mitotic timing and reduces the number of cells entering mitosis **(****Figure 9A and B****), C2** treatment does not increase the proportion of cells with multipolar spindle, unlike what has been observed in 786-O cells **(****Figure 9E****).** Moreover, no major cell death was observed in interphase (1.5% vs. 0% in CTRL), during mitosis (2% vs. 0% in CTRL) or after mitotic exit in the ensuing interphase (1% vs. 0% in CTRL) **(****Figure 9C** and **D****),** compared to 786-O.

### Example 8: Specific efficacy of a drug combination according to the invention in cells with abnormal centrosome numbers

Based on results presented above, it was hypothesized that one of the main mechanisms by which **C2** kills cancer cells is by preventing spindle pole clustering. To validate this hypothesis and test whether **C2** can also target other carcinoma cells with elevated centrosomes (>2) or centriole (>4) numbers, its effects was evaluated on two melanoma cell lines that have been reported to either have low (M14) (5 % of extra centrioles) or abnormally high (MDA-MB-435 or MDA435) (45 % of extra centrioles) levels of abnormal centrosome numbers **(****Figure 10A** and **B****)** (*Marteil et al., 2018, supra*)*.* Cell viability and live cell imaging experiments as done for previous ccRCC cell lines were used to assess C2 effects on M14 and MDA435. Interestingly, **C2** inhibited cell viability much more efficiently in MDA435 than in M14 cells (90 ± 10%, vs. 40 ± 11%; **Figure 10C**). Live cell imaging indicated that the **C2-**treatment resulted in a mitotic timing delay in MDA435 cells **(****Figure 10D** and **E****),** centrosome segregation defects, a higher incidence of death in mitosis **(****Figure 10G**) and a strong impairment of spindle pole clustering **(****Figure 10H****).** In contrast, in M14 cells, the only visible effect after **C2-**treatement was a mild delay in mitotic timing **(****Figure 10D-G**).
Those data supports that **C2** selectively targeted MDA435 cells possessing abnormally high numbers of centrioles (more than 4) and that, in contrast, M14 cells with mostly normal centrosome/centriole numbers, were less affected by **C2.** This implies that **C2** might be particularly effective against cells with abnormal number of centrioles/centrosomes since the data suggest that **C2** generally increases the propensity of cells to form multipolar spindles, as it increased the percentage of cells with multipolar spindles at mitotic onset in 786-O, 786-OsunR and MDA435 cells.
Finally, in 786-O and 786-OsunR cells, **C2** triggered cell death both in interphase and in mitosis **(****Figure 11****)** which supports that **C2** also targets cancer cells via a mitosis-independent mechanism. Several declustering agents have been shown to target cells both in interphase and mitosis, yet their mechanism of action focuses on the disruption of the nuclear-centrosome-golgi axis, which inhibits cell migration inhibition (Pannu et al., 2014, Cell death & disease, 5, e1538, doi:10.1038/cddis.2014.505). Since **C2** did not affect cell migration, **C2** seems to have a different mechanism of action in interphase.

Altogether those data support the robustness of a method of the invention. It is worth mentioning that when analysing the regression models derived from the screen under step f), the generated first-order terms clearly confirmed differences in mechanism and selectivity of the different drug classes included in the screen, i.e. HDACI and KI. While HDACI tended to have negative regression coefficients for efficacy in the cancer and non-cancerous cells, indicating a lack of selectivity towards malignant cells, KI generally gave positive regression coefficients in the TW, indicating selective activity in cancer cells. Therefore, using the TW as an objective parameter for optimization, together with constraints on the compound doses limited by the maximally attainable clinical plasma drug concentrations are important features of a method of the invention.
Further, the last step of the method, i.e. the dose optimization step using both drug activity and the TW, advantageously allowed for further increased efficacy and selectivity of the identified compound combination.
Those data further support that an active selection of a phenotype to be targeted (in particular the viability of cancer cell) allows to identify a highly efficient and selective drug combination which is optimized for cell viability inhibition but which did not necessarily present enhanced activity in the cell migration inhibition or endothelial network formation although it did show anti-angiogenic properties when tested in a developmental angiogenesis assay (i.e. the chicken embryo chorioallantoic membrane assay).

Therefore, a method of the invention represents a powerful tool for identifying low-dose multi-drug combinations with minimal activity in cross-validated non-malignant cells. In particular, the potential of this method was illustrated by the identification of a four-compound combination which retained its anti-cancer efficacy in 786-OsunR cells, thus evidencing its potential to overcome cross-resistance mechanisms.

The ability of the identified combination in preventing the clustering of multipolar spindles in 786-O cells and MDA435 cells was unexpected and the properties of an identified combination **C2** are very promising since compounds that prevent pole clustering have emerged as a promising therapeutic agent, since they specifically target cancer cells that form multi-polar spindles due to abnormal centrosome numbers, but do not affect mitotic progression in non-malignant cells with normal centrosome numbers. Indeed, while normal cells as a rule contain at mitotic onset 2 centrosomes with 2 centrioles, cancer cells frequently display abnormalities in centrosome organization, such as supplementary centrosome numbers (*Godinho et al., 2014, supra*)*.*

## Claims

1. An method for identifying low-dose multi-drug combinations for the treatment of cancer, in particular solid tumor cancers, said method comprising the steps of:
a) providing separately a set of cancer cells (malignant) and a set of non-cancer cells (non-malignant);
b) selecting an initial set of candidate compounds to be assayed;
c) contacting said set of cancer cells and a set of non-cancer cells with each of the selected candidate compound and measuring metabolic activity of said cells for different concentrations of candidate compound thereby generating dose-response curves for each candidate compound;
d) designing a candidate compound combination matrix by orthogonal array composite design (OACD);
e) contacting a new set of cancer cells and a new set of non-cancer cells with each of the candidate compound combinations designed under step d) and measuring the difference in anti-cancer efficacy measured by a selected high-throughput assay (such as cell growth or viability inhibition, induction of cell death or apoptosis, inhibition of cell migration, tumor growth inhibition, etc.) on each cells sets for each candidate compound combination, thereby defining a therapeutic window for each compound candidate compound combination;
f) Analysing the anti-cancer efficacy of each candidate compound in the defined therapeutic window and determining the contribution of each candidate compound as an individual agent and as a candidate compound pair to the overall activity of each of the candidate compound combinations generated under step d) by a second-order linear regression analysis;
g) Eliminating compounds from the candidate compound combinations based on a lack of synergistic interaction and/or no statistical significance in single compound first order terms in the second-order linear regression analysis model;
h) Reiterating steps d) to g) [please indicate how many iterations are needed at min/max and when one knows to stop iterating] by including in a new matrix under step d), candidate compounds at specified dosage levels and selecting under g) candidate compound combinations which show desirable compound interactions and result in sufficient inhibition of the targeted cell process selected in e);
i) Optimizing concentrations of compounds of the candidate compound combinations identified at the end of step h) after two to three iterations, by expanding the range of compound doses considered up to the maximum compound plasma concentration achievable in patients, including more concentrations values for each compound and exploring experimentally testing higher resolution design matrices and applying a regression model to identify the optimal compound concentration combination can be predicted as optimal solution of the regression model;
j) Identifying a low-dose candidate compound combination with the highest anti-cancer activity with minimal toxicity assessed under step g)

2. A method according to claim 1, wherein malignant cells are clear cell renal cell carcinoma (ccRCC cell) (e.g. 786-O).

3. A method according to claim 1 or 2, wherein non-malignant cells are from the same origin as the cancer of interest or other cells representing quantifiable indicators of toxicity.

4. A method according to any one of claims 1 to 3, wherein non-malignant cells are human embryonic kidney 293 cells (HEK-293T).

5. A low-dose multi-drug combination identified by a method according to any one of claims 1 to 4.

6. A low-dose multi-drug combination according to claim 5 for use in the treatment of cancer.

7. A combination of CI-994, tubacin, erlotinib and dasatinib for use in the treatment of a cancer.

8. A combination for use according to claim 7, wherein CI-994 is at a concentration of 5 µM), tubacin is at a concentration of 5 µM), erlotinib is at a concentration of 5 µM and dasatinib at a concentration of 6.3 µM.

9. A combination for use according to any one of claims 6 to 8 wherein said cancer presents cells with centrosome clustering.

10. A combination for use according to any one of claims 6 to 9, wherein said cancer presents a high percentage of cells prone to form multipolar spindles due to elevated centrosome number, centriole breakages, pre-mature centriole dis-engagement, or pericentriolar material fragmentation.

11. A combination for use according to any one of claims 6 to 10, wherein said cancer is selected from breast, ovaries, colon, brain, prostate, kidney, skin and lung cancers.

12. A combination for use according to any one of claims 6 to 11, wherein said cancer is melanoma.

13. A pharmaceutical composition comprising a combination of CI-994, tubacin, erlotinib (e.g. erlotinib hydrochloride) and dasatinib and at least one pharmaceutically acceptable carrier.

14. A pharmaceutical composition according to claim 13, wherein CI-994 is at a concentration of 5 µM, preferably at a concentration of 2.5 µM, tubacin is a concentration of 5 µM, erlotinib is at a concentration of 5 µM and dasatinib at a concentration of 6.3 µM.

15. A pharmaceutical composition according to claim 13 or 14 for use as a medicament.
